# EUROPEAN PATENT APPLICATION

(11) **EP 1 997 905 A1**
(43) Date of publication of application: **03.12.2008**
(21) Application number: 07109429.6
(22) Date of filing: 01.06.2007
(51) Int. Cl.: C12Q 1/68

(54) **Nucleic acid amplification**

(71) Applicant: Friesland Brands B.V., 7943 PE Meppel (NL)
(72) Inventor: Nauta, Arjen, 6721 CG Bennekom (NL); van den Heuvel, Elisabeth, Gertruda Hendrika, Maria, 3572 GS Utrecht (NL); Veurink, Janine, Henriët, 7681 SK Vroomshoop (NL); Geurts, Johannes, Marie, Wilhelmus, 7559 NN Hengelo (NL)
(74) Representative: van Loon, C.J.J.

(57) **Abstract**

The invention provides means and methods for amplifying and/or detecting nucleic acid sequences of different genera and/or species. Particularly, a plurality of microbial nucleic acid sequences are amplified. Primers, probes and kits are also provided as well as means and methods for screening for candidate probiotics and prebiotics.

## Description

The invention relates to the fields of biology and microbiology.

Detection and isolation of nucleic acid is commonly performed in a wide variety of applications. Nucleic acid is for instance detected in order to determine the presence and/or identity of a micro-organism of interest. In various diagnostic applications samples are screened for the presence of nucleic acid of a micro-organism of interest such as a pathogen in order to determine whether an individual is infected. The presence, amount and/or identity of multiple micro-organisms of a heterogeneous population, such as the population of for instance the gastro-intestinal tract, an environmental sample or a starter culture, is also determined by investigating microbial nucleic acid present in a sample obtained from such population. Instead of determining whether one specific micro-organism is present, the presence and/or amount of a variety of micro-organisms is often determined. The health state of an individual is for instance examined by determining whether a favourable equilibrium exists between various micro-organisms present in a sample from said individual, preferably derived from the gastro-intestinal tract. Since the gastro-intestinal tract houses a wide variety of bacterial species and genera, the presence and/or amount of a plurality of micro-organisms is often determined in order to assess the status of an individual. A favourable equilibrium is indicative for a healthy status. For instance, significant amounts of bifidobacteria and lactobacilli are preferably present in the gastro-intestinal tract as they are associated with resistance to gastroenterological infections and resistance to tumors such as colon cancer. Overgrowth of harmful species, such as for instance clostridia, staphylococci, pseudomonas and/or klebsiella, as well as limited presence of beneficial species such as bifidobacteria and lactobacilli results in a less favourable health state or even disease. Hence, the normal equilibrium between micro-organisms in the gastro-intestinal tract is important for the well-being of individuals. Disturbance of the healthy equilibrium, for instance as a result of immune depression, disease, stress, or the use of antibiotics, other drugs or excess alcohol, decreases an individual's well-being. The bacterial flora also alters with increasing age. Lower numbers of bifidobacteria and lactobacilli are present in the bacterial flora in human individuals over 60 years of age, compared to younger healthy adults, and the amount of clostridia is increased. These bacteriological changes are considered to have an influence on the general health status and often coincide with an increase in susceptibility to gastroenterological infections and chronic diseases like colon cancer.

Also in livestock, pathological conditions have been observed that do not seem to be attributable to an infection with a single pathogen, but which merely result from the distortion of a healthy equilibrium which normally exists within the different micro-organisms constituting the bacterial flora. Examples of conditions related to a lack of healthy equilibrium are steathorroea in calves and ileitis in pigs. Distortion of a normal equilibrium is for instance caused by the feed intake of animals, by immune depression and/or environmental effects such as temperature and humidity.

Other examples of complex microbial populations are populations present in starter cultures, environmental samples and (fermented) food samples. The composition of such populations is for instance investigated during food quality control or pollution control. The presence of micro-organisms in a sample is often demonstrated by detecting nucleic acid derived from said micro-organisms, for instance with a labelled probe. Said nucleic acid is often amplified in order to lower the detection limit. If the presence of a specific micro-organism such as a pathogen is investigated one specific nucleic acid sequence is often amplified and detected. This is usually done using specific primers and probes. However, if amplification of a wide variety of nucleic acids is desired, specific primers are less suitable in view of the large numbers of primers needed. The use of a common primer capable of annealing to nucleic acid sequences of various species and/or genera is preferred. Multiple primer pairs with one common primer and several genus-specific primers are preferably used. It is however not always possible to combine a plurality of genus-specific primers with one common primer because the sequences of a primer pair are chosen such that the length of the nucleic acid sequence that is to be amplified is at most 300 base pairs, otherwise the efficiency of amplification is not optimal for quantitative nucleic acid amplification protocols. The locations of genus-specific sequences vary within the genomes of different organisms. As a result, genus-specific primers are used in the art which anneal at different sites. It is not always possible to use primer pairs wherein various different specific primers are each combined with one common primer, because the length of the amplified nucleic acid sequences is often too large. Therefore, if a wide variety of genera and species is to be amplified, which is for instance the case if a sample of a complex ecosystem, an environmental sample or a biological sample is investigated, many different primer pairs are currently used. This involves amongst other things the disadvantage that the number of different primer sequences used in the same reaction assay is large. Since the amount of different primers that is capable of being efficiently used in one reaction assay is limited, it is not possible to efficiently amplify all nucleic acid sequences when the number of different primers is too large. Therefore, separate amplification reactions are commonly performed if a large number of different nucleic acid sequences is to be amplified. Separate amplification reactions involve less accurate comparisons between the results because small differences between the reaction conditions of the separate assays, such as for instance temperature, pH and the amount of primers, influences the results of the assays. This is particularly of relevance if nucleic acid is quantified.

Another disadvantage of currently used methods is that amplification reactions are often not specific enough (meaning that nucleic acid other than a nucleic acid sequence of interest is amplified or detected, often resulting in false-positive results) or too specific (meaning that not all kinds of nucleic acids of interest are amplified or detected so that false negative results are obtained). A non-limiting example of a false negative result is the non-observance of a certain strain of a species of interest.

It is an object of the present invention to provide improved methods for amplifying and/or detecting nucleic acid sequences. Preferably, means and methods for amplifying and/or detecting nucleic acid sequences of different genera and/or species are provided.

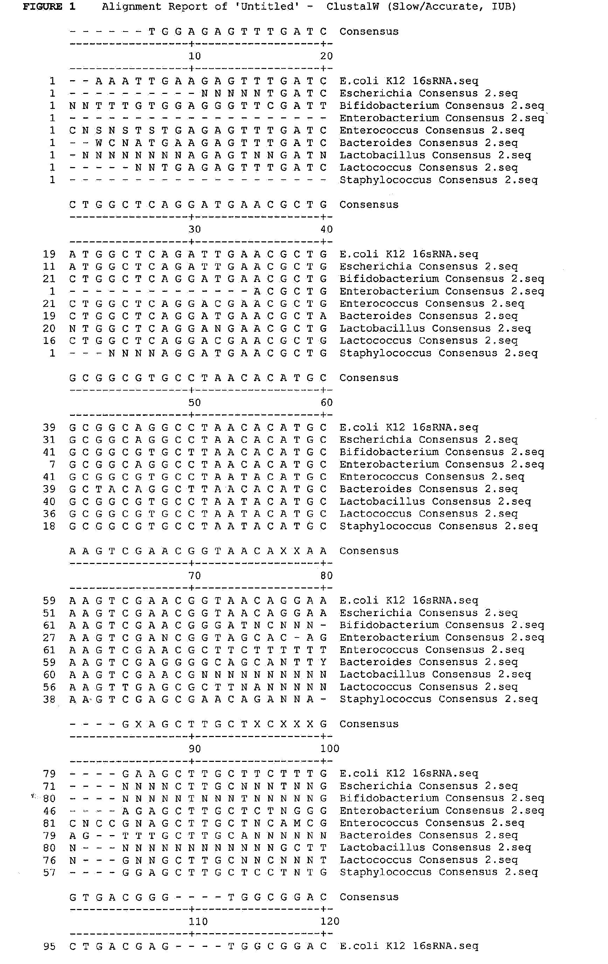

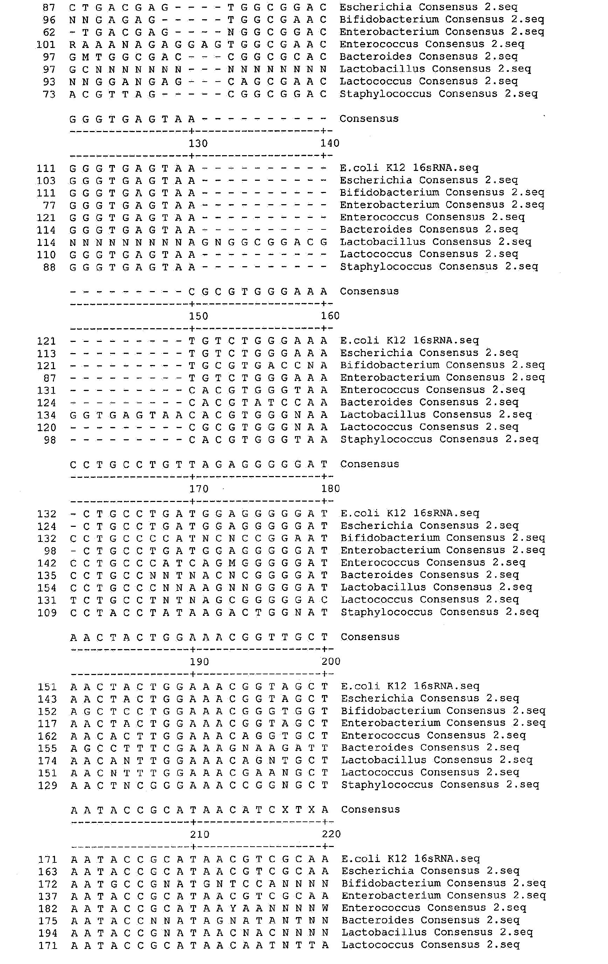

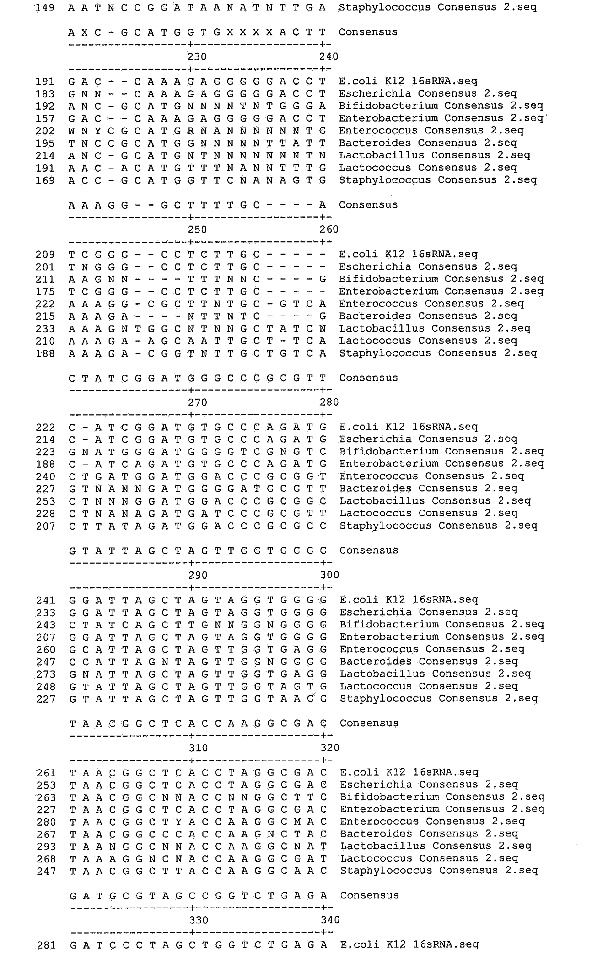

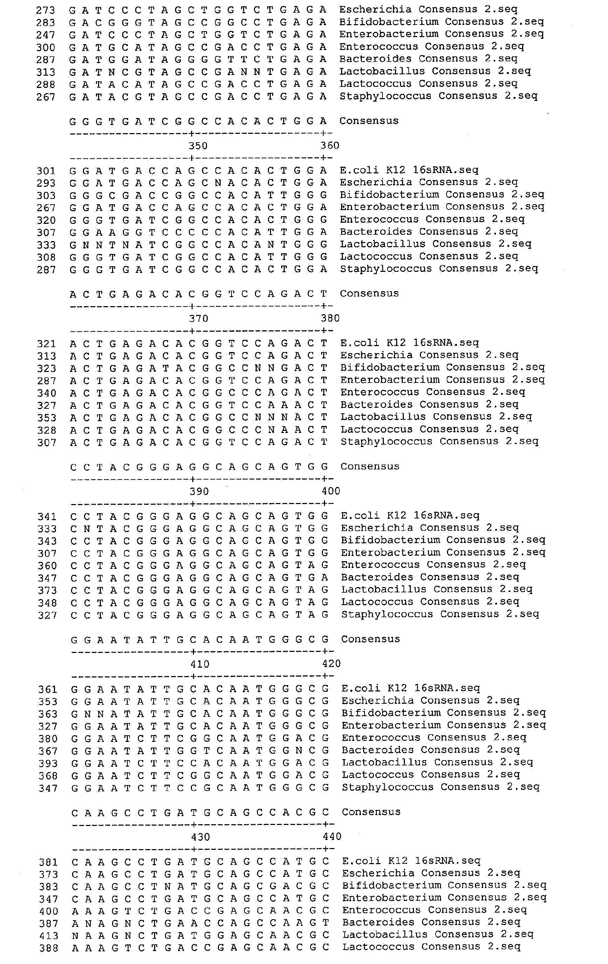

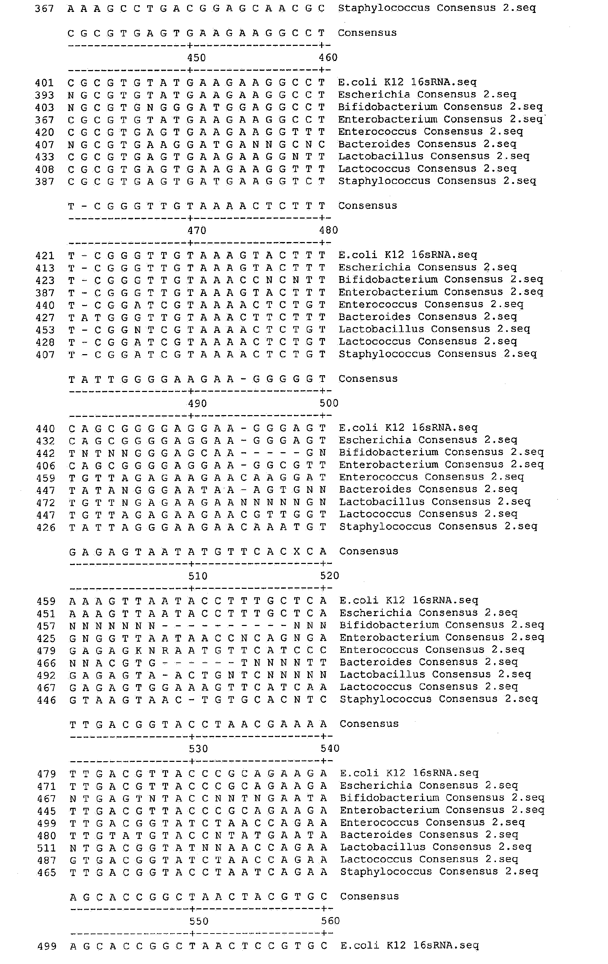

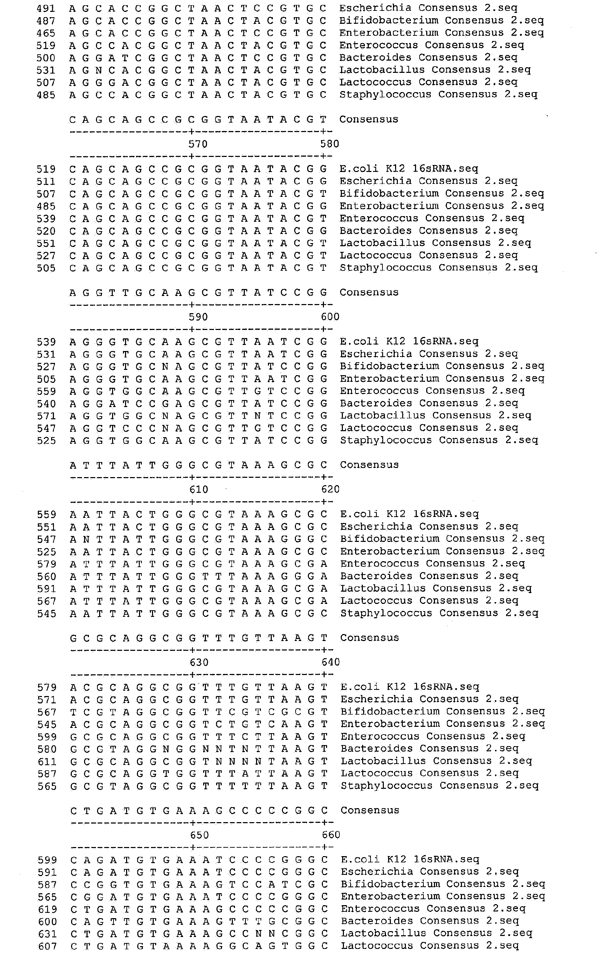

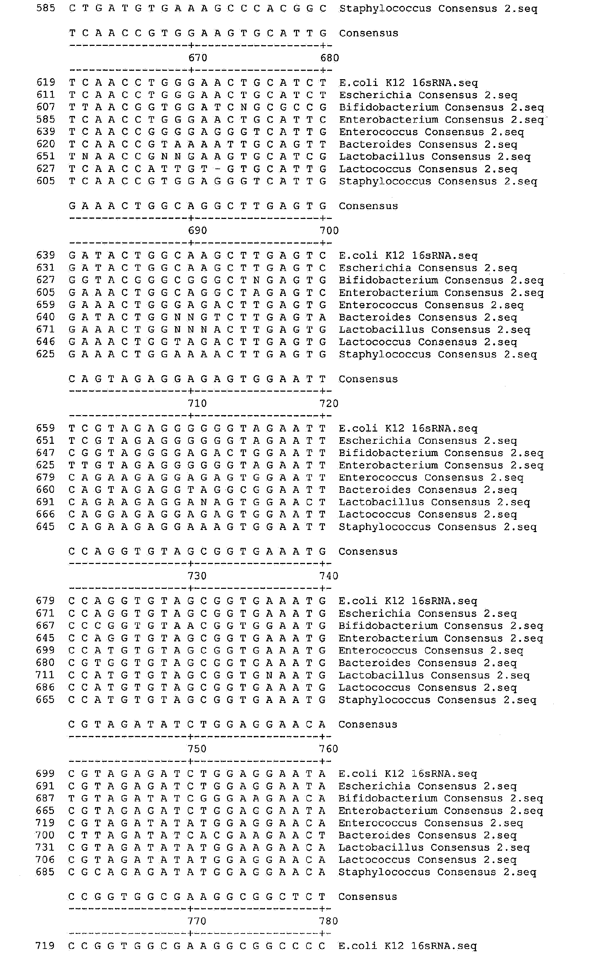

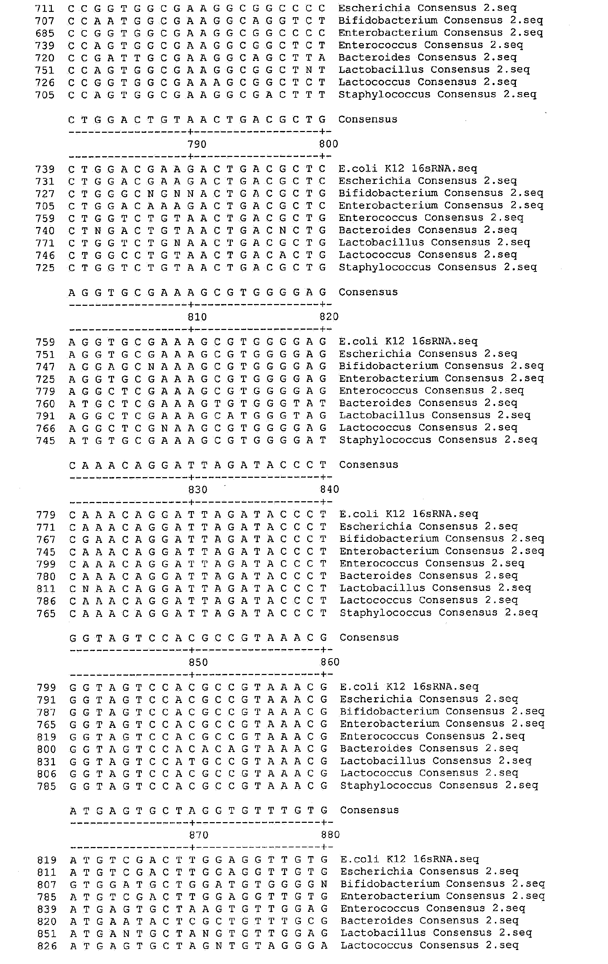

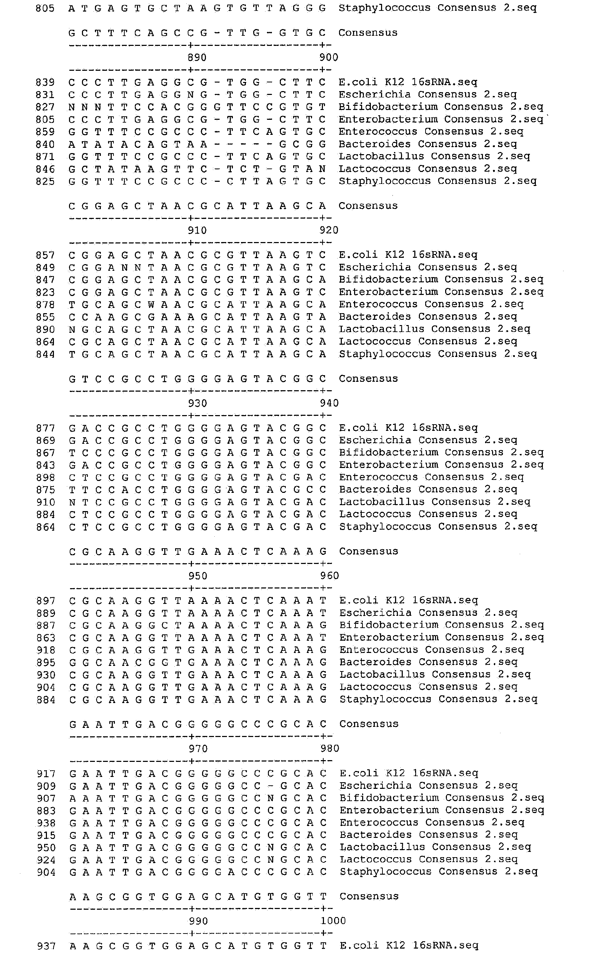

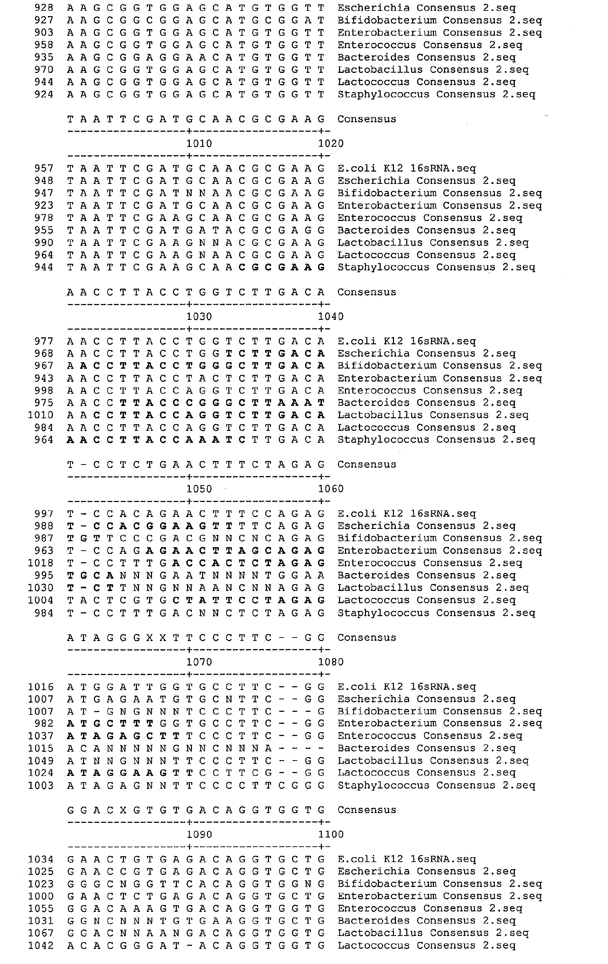

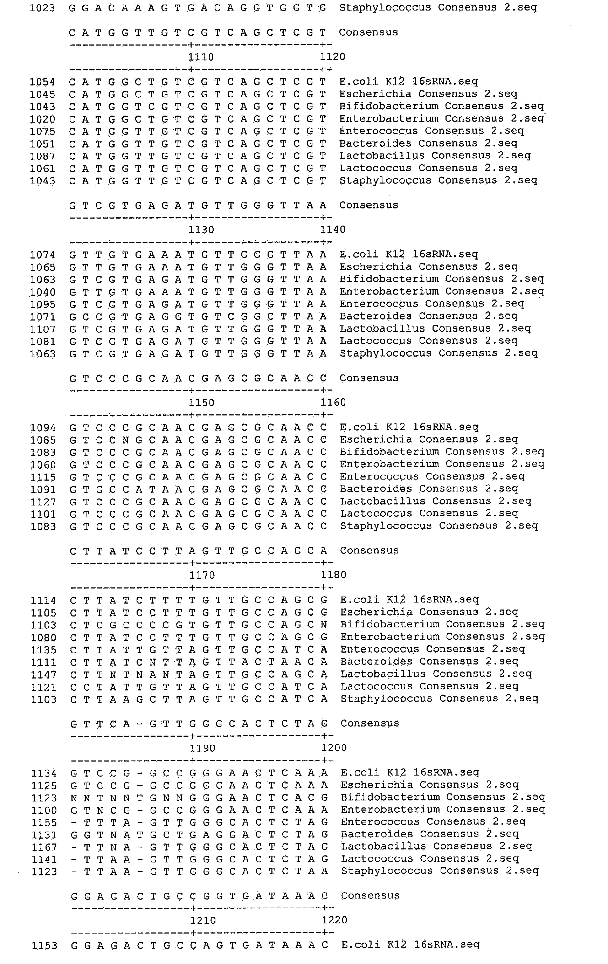

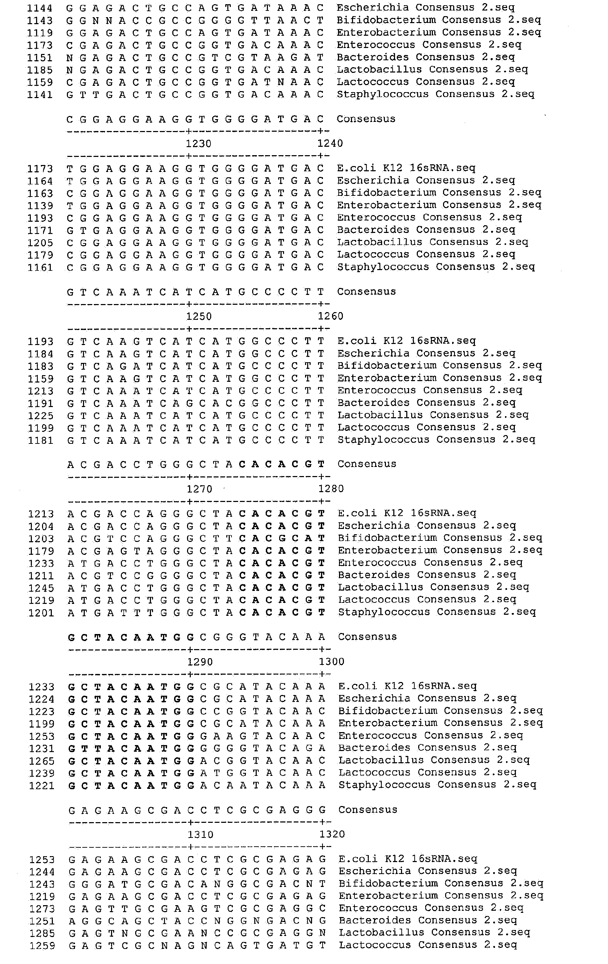

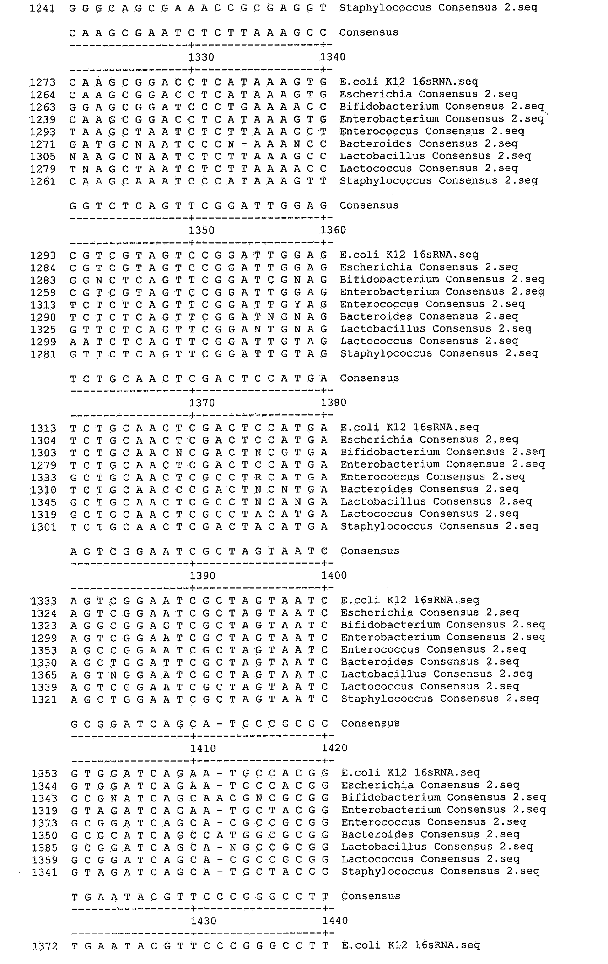

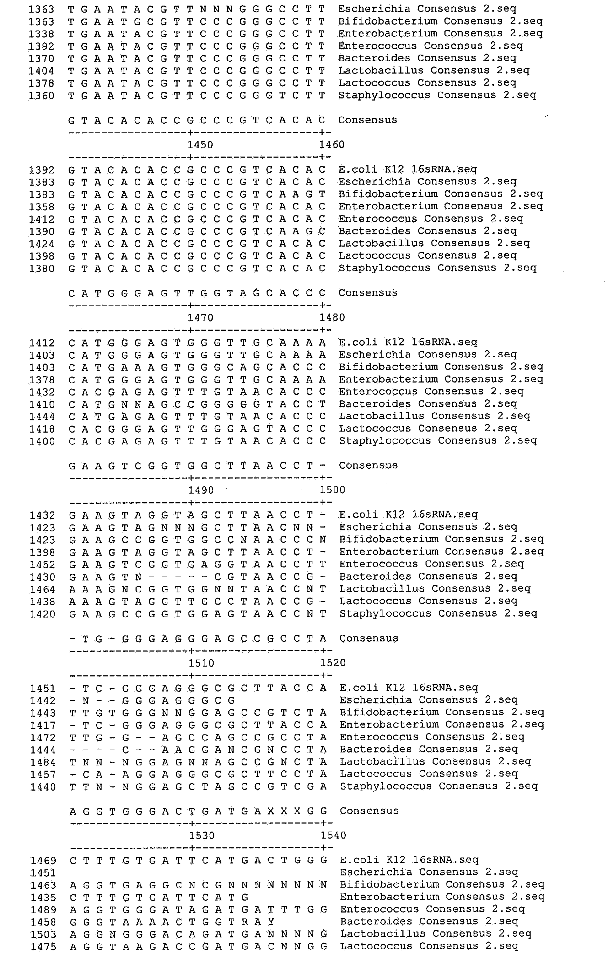

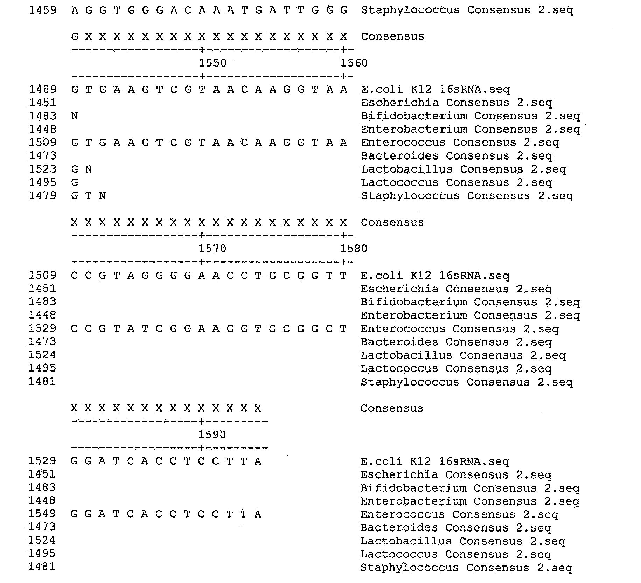

The invention provides a method wherein one common primer, capable of annealing to nucleic acid of at least eight different bacterial genera, is used in order to amplify nucleic acid of interest in a sample. The invention provides the insight that a primer with a length of between 9 and 50 nucleotides, preferably with a length of between 9 and 40 nucleotides, more preferably with a length of between 15 and 25 nucleotides, which is capable of specifically binding to a 16S rRNA nucleic acid sequence corresponding to the nucleic acid sequence located from about nucleotide position 1226 to about 1242 in the 16S rDNA sequence of Escherichia coli K12 as depicted in Figure 1, said positions being indicated in Figure 1, is particularly suitable for amplifying nucleic acid. Using sequence alignments, a skilled person is well capable of determining which 16S rRNA nucleic acid sequences of other genera, species and strains correspond to said 16S rDNA sequence of Escherichia coli K12 as depicted in Figure 1. Figure 1 provides an alignment but, of course, alignments with 16S rRNA/rDNA sequences of other micro-organisms are possible.

A primer according to the invention is preferably used for amplifying at least two, preferably at least three, more preferably at least four, more preferably at least five, more preferably at least six, more preferably at least seven, most preferably at least eight bacterial genera. A common primer according to the invention is preferably combined with genus-specific primers in order to specifically amplify nucleic acid of interest. The genus-specific primers are preferably capable of annealing to a site within a bacterial genome which is less than 300 nucleotides located from the annealing site of a common primer according to the invention. This way, the efficiency of nucleic acid amplification is optimal for quantitative amplification protocols. It has now become possible to generate a plurality of primer pairs containing a common primer according to the present invention and a second, genus-specific primer, wherein the resulting amplified nucleic acid of each genus has a length of 300 nucleotides or less. This means that the efficiency of nucleic acid amplification is optimal for quantitative amplification protocols. In a preferred embodiment each primer pair comprises one common primer with the same sequence. This significantly reduces the amount of different primer sequences.

In one preferred embodiment a method according to the invention is performed with at least four different primer pairs according to the invention, each primer pair comprising the same common primer according to the invention and a different genus-specific primer. Preferably, at least five different primer pairs according to the invention are used. More preferably, at least six, more preferably at least seven different primer pairs according to the invention are used. Most preferably, at least eight different primer pairs according to the invention are used. The more primer pairs according to the invention are used, the more genera can be detected, if present. Preferably, all primer pairs according to the invention have an identical common primer according to the invention and a different genus-specific primer. The primer pairs according to the invention are preferably used for amplifying a genus-specific nucleic acid sequence of between 20 and 300 nucleotides, preferably between 50 and 200 nucleotides. Contrary to current methods in the art, a common primer has now been provided which allows for the design of at least eight, preferably at least ten different genus-specific primer pairs which have an identical common primer and which are capable of amplifying at least eight different genus-specific nucleic acid sequences with a length of between 20 and 300 nucleotides.

One aspect of the invention therefore provides a method for subjecting a sample to a nucleic acid amplification reaction comprising providing said sample with at least one primer and performing a nucleic acid amplification reaction, wherein said sample is provided with a primer with a length of between 9 and 50 nucleotides, preferably with a length of between 9 and 40 nucleotides, more preferably with a length of between 15 and 25 nucleotides, which is capable of specifically binding to a 16S rRNA nucleic acid sequence corresponding to the nucleic acid sequence located from about nucleotide position 1226 to about 1242 in the 16S rDNA sequence of Escherichia coli K12 as depicted in Figure 1, said positions being indicated in Figure 1. Said primer is preferably capable of specifically binding to a nucleic acid sequence located from about nucleotide position 1274 to about 1290 in the general consensus sequence depicted in Figure 1, and/or to a nucleic acid sequence located from about nucleotide position 1216 to about 1232 in the 16S rDNA sequence of the Bifidobacterium consensus 2 sequence depicted in Figure 1, and/or to a nucleic acid sequence located from about nucleotide position 1224 to about 1240 in the 16S rDNA sequence of the Bacteroides consensus 2 sequence depicted in Figure 1, said positions being indicated in Figure 1.

Ribosomal RNA (rRNA) is the central component of the ribosome, the protein manufacturing machinery of all living cells. The small 30S subunit of prokaryotic ribosomes comprises 16S rRNA and various proteins. The 16S rRNA is important for subunit association and translational accuracy. Figure 1 shows an alignment of (consensus) 16S rDNA sequences of eight bacterial genera. Since the 16S rDNA sequences are highly conserved, the corresponding positions of 16S rDNA sequences in other micro-organisms are easily assessed based on homology. For instance, nucleotide positions 1226 to 1242 in the 16S rDNA sequence of Escherichia coli K12 as depicted in Figure 1 correspond to nucleotide positions 1216 to 1632 in the consensus 16S rDNA sequence of Bifidobacteria depicted in Figure 1. According to the present invention, a primer capable of specifically binding to a nucleic acid sequence at a location in a 16S rRNA nucleic acid sequence which corresponds to nucleotide position 1274 to 1290 in the general 16S rDNA consensus sequence as depicted in Figure 1 is particularly suitable for amplification of a wide variety of bacterial genera. Said primer is preferably combined with at least two, preferably at least three, more preferably at least four, more preferably at least five, more preferably at least six, more preferably at least seven, most preferably at least eight genus-specific primers. In a preferred embodiment a common primer according to the invention is used which is capable of specifically binding to a nucleic acid sequence located from about nucleotide position 1274 to about 1290 in the general consensus sequence depicted in Figure 1. Said primer is preferably capable of specifically binding to said nucleic acid sequence under stringent conditions. Using stringent conditions, unspecific annealing of primers is best avoided.

A 16S rRNA nucleic acid sequence is defined herein as any kind of (natural or non-natural) nucleic acid sequence corresponding to a 16S rRNA sequence. A nucleic acid sequence corresponds to a 16S rRNA sequence when said nucleic acid sequence comprises a sequence which is capable of specifically binding to a complementary sequence of said 16S rRNA sequence. A nucleic acid sequence also corresponds to a 16S rRNA sequence when said nucleic acid sequence comprises a sequence which is capable of specifically binding to a 16S rRNA sequence, meaning that said nucleic acid sequence comprises a sequence which is complementary to said 16S rRNA sequence. The term "16S rRNA nucleic acid sequence" for instance encompasses a DNA sequence comprising a sequence which is capable of specifically binding to a 16S rRNA sequence. The term "16S rRNA nucleic acid sequence" also encompasses a DNA sequence comprising a sequence which is identical to a 16S rRNA sequence (except, of course, the fact that DNA comprises thymine instead of uracil). The term "16S rRNA nucleic acid sequence" hence encompasses a 16S rDNA sequence. As used herein, the term "16S rRNA sequence" means any kind of 16S rRNA nucleic acid sequence.

A first nucleic acid molecule specifically hybridizes to a second nucleic acid molecule if said first nucleic acid molecule comprises a sequence of at least four, preferably at least five, more preferably at least six nucleotides which is complementary to at least part of a sequence of said second nucleic acid molecule. As is well known by the skilled person, an adenine (A) is complementary to a thymine (T) or uracil (U). A guanine is complementary to a cytosine (C). The term "specifically hybridizing" or "specifically binding" of a nucleic acid sequence to another nucleic acid sequence therefore means a binding event resulting from hybridization of complementary nucleic acid sequences. The terms do not encompass non-specific sticking of nucleic acid molecules. However, under mild conditions two nucleic acid molecules may bind which comprise sequences that are partly complementary to each other. Hence, nucleic acid sequences that are less than 100% complementary to each other may nevertheless bind under mild conditions such as a low temperature. As used herein, two nucleic acid sequences specifically bind each other if their sequences are at least 70%, preferably at least 75%, more preferably at least 80% complementary to each other.

If stringent conditions are used, two nucleic acid molecules bind each other if they comprise a sequence which is at least 85%, preferably at least 90% complementary to a sequence of the other nucleic acid molecule. Stringent conditions are therefore defined herein as conditions where hybridization of nucleic acid molecules only takes place if said nucleic acid molecules comprise a sequence which is at least 85%, preferably at least 90% complementary to a sequence of the other nucleic acid molecule.

Since the same common primer according to the invention is suitable for use in each primer pair, it has become possible to reduce the number of different primers when amplifying a plurality of different nucleic acid sequences of interest in the same assay. This is for instance illustrated as follows. If nucleic acid of four different genera is to be specifically amplified using current methods known in the art, four specific primer pairs would be required, said four primer pairs comprising four genus-specific forward primers and four genus-specific reverse primers. Hence, eight different primers are needed. With a method according to the present invention it has become possible to use one identical common primer, capable of annealing to nucleic acid of the four different genera, and four additional genus-specific primers. Hence, only five different primers are needed instead of eight. Likewise, if nucleic acid of eight different genera is to be amplified, currently used primer pairs contain eight genus-specific forward primers and eight genus-specific reverse primers. Hence, sixteen different primers are needed. A method according to the present invention however only needs one common primer capable of annealing to nucleic acid of the eight genera and eight genus-specific primers. Hence, only nine different primers are needed instead of sixteen.

With current methods this approach was not possible. Different forward primers as well as different reverse primers were needed because at least eight different primer pairs comprising the same common primer and eight specific primers, wherein all primer pairs are capable of specifically amplifying genus-specific nucleic acid with a length of between 20 and 300 nucleotides, were not available before the present invention. Therefore, using current methods, the required number of different primers was higher than the number of different primers which is used with a method according to the present invention. An advantage of a method according to the invention is the fact that more different kinds of species are detectable in one assay because a lower number of different primer sequences and/or probe sequences is needed. Use of a lower number of different primer sequences and/or probe sequences also reduces costs.

A common primer according to the present invention is defined as a primer with a length of between 9 and 50 nucleotides, preferably with a length of between 9 and 40 nucleotides, more preferably with a length of between 15 and 25 nucleotides, which is capable of specifically binding nucleic acid sequences of at least eight genera, preferably bacterial genera, wherein said primer is capable of specifically binding to a 16S rRNA nucleic acid sequence corresponding to the nucleic acid sequence located from about nucleotide position 1226 to about 1242 in the 16S rRNA sequence of Escherichia coli K12 as depicted in Figure 1, said positions being indicated in figure 1.

A nucleic acid amplification reaction comprises any kind of reaction wherein a nucleic acid sequence is duplicated at least one time. Preferred amplification reactions comprise (reverse transcriptase)-polymerase chain reaction ((RT)-PCR), nucleic acid sequence based amplification (NASBA), strand displacement amplification (SDA) and transcription mediated amplification (SDA). Methods of performing a nucleic acid amplification reaction are well known in the art. A PCR protocol is for instance described in (Sambrook, J., Fritsch, E.F., and Maniatis, T. 1989. Molecular cloning: a laboratory manual. Cold Spring Harbor laboratory Press, Cold Spring Harbor, N.Y.). (Called herein Sambrook et al (1989)).

A nucleic acid sequence as used herein for instance comprises naturally occurring nucleic acids, such as DNA, RNA or a DNA/RNA helix, modified nucleic acid sequences and/or nucleic acid derivatives such as for instance peptide nucleic acid (PNA). Possible bases of a nucleic acid sequence according to the invention comprise naturally occurring purines and/or pyrimidines, such as adenine, thymine, cytosine, guanine and uracil, as well as modified bases such as for instance inosine.

A method according to the invention is particularly suitable for amplifying nucleic acid of Bifidobacteria, Bacteroides, Enterococci, Enterobacteria, Staphylococci, Lactobacilli, Lactococci and/or Escherichia. The positions within the 16S rRNA nucleic acid sequence of each of these genera, as compared to the 16S rDNA sequence of Escherichia coli K12, are depicted in Figure 1. A common primer according to the present invention is preferably capable of specifically binding to a nucleic acid sequence located from about nucleotide position 1216 to about 1232 in the consensus 16S rDNA sequence of Bifidobacteria (Bifidobacterium consensus 2 sequence depicted in Figure 1), to a nucleic acid sequence located from about nucleotide position 1224 to about 1240 in the consensus 16S rDNA sequence of Bacteroides (Bacteroides consensus 2 sequence depicted in Figure 1), to a nucleic acid sequence located from about nucleotide position 1246 to about 1262 in the consensus 16S rDNA sequence of Enterococci (Enterococcus consensus 2 sequence depicted in Figure 1), to a nucleic acid sequence located from about nucleotide position 1192 to about 1208 in the consensus 16S rDNA sequence of Enterobacteria (Enterobacterium consensus 2 sequence depicted in Figure 1), to a nucleic acid sequence located from about nucleotide position 1214 to about 1230 in the consensus 16S rDNA sequence of Staphylococci (Staphylococcus consensus 2 sequence depicted in Figure 1), to a nucleic acid sequence located from about nucleotide position 1258 to about 1274 in the consensus 16S rDNA sequence of Lactobacilli (Lactobacillus consensus 2 sequence depicted in Figure 1), to a nucleic acid sequence located from about nucleotide position 1232 to about 1248 in the consensus 16S rDNA sequence of Lactococci (Lactococcus consensus 2 sequence depicted in Figure 1), and/or to a nucleic acid sequence located from about nucleotide position 1217 to about 1233 in the consensus 16S rDNA sequence of Escherichia coli (Escherichia consensus 2 sequence depicted in Figure 1). A common primer according to the invention is capable of annealing to a wide variety of species of each of said genera, as for instance illustrated in Example 1. Hence, a primer pair comprising said common primer according to the invention and a genus-specific primer which is specific for at least one of said genera is capable of amplifying nucleic acid of many species and/or strains of said genus. False-positive results (due to lack of specificity) and false-negative results (due to lack of amplification of a species/strain of the same genus) are diminished. Further provided is therefore a method according to the invention, wherein said sample comprises nucleic acid of at least one member of Bifidobacteria, Bacteroides, Enterococci, Enterobacteria, Staphylococci, Lactobacilli, Lactococci and/or Escherichia. This embodiment is for instance particularly suitable for determining the presence and/or (relative) amounts of at least one of these genera in a sample derived from the gastrointestinal tract, in order to determine whether a healthy equilibrium is present in the intestinal flora. Preferably said sample comprises nucleic acid of at least one member of Bifidobacteria, Bacteroides, Enterococci, Enterobacteria, Staphylococci, Lactobacilli, Lactococci and Escherichia.

In a particularly preferred embodiment a common primer according to the invention is used which has a length of between 10 and 50 nucleotides, preferably a length of between 10 and 30 nucleotides, and which is at least 80% homologous to at least part of the sequence 5' CCA TTG TAR CAY GYG TGW AG 3'. As used herein, R stands for a purine (G or A), Y stands for a pyrimidine (T or U or C) and W stands for an A or a T or a U. Said part preferably has a length of at least 15 nucleotides, more preferably at least 18 nucleotides. Such primer is particularly suitable for amplifying nucleic acid of at least one member of Bifidobacteria, Bacteroides, Enterococci, Enterobacteria, Staphylococci, Lactobacilli, Lactococci and/or Escherichia. Further provided is therefore a method according to the invention, comprising providing a sample with a primer with a length of between 10 and 50 nucleotides, preferably with a length of between 10 and 30 nucleotides comprising a sequence which is at least 80% homologous to at least part of the sequence 5' CCA TTG TAR CAY GYG TGW AG 3', said part having at least 15 nucleotides, preferably at least 18 nucleotides. Possible modifications to the primer sequence 5' CCA TTG TAR CAY GYG TGW AG 3' resulting in a homologous sequence for instance comprise deletion, alteration, substitution or addition of between 1 and 5 nucleotides. Deletion of a primer nucleotide is preferably performed at the 5' end of said primer because elongation during nucleic acid amplification takes place at the 3' end. If nucleotide substitution is performed, adenine to thymine substitution is preferred over cytosine to guanine substitution.

In a preferred embodiment said primer is at least 85% homologous to at least part of the sequence 5' CCA TTG TAR CAY GYG TGW AG 3', said part having at least 15 nucleotides, preferably at least 18 nucleotides. More preferably, said primer is at least 90%, more preferably 95%, more preferably 97%, most preferably 98% homologous to at least part of the sequence 5' CCA TTG TAR CAY GYG TGW AG 3', said part having at least 15 nucleotides, preferably at least 18 nucleotides. The higher the homology, the more the primer sequence resembles the sequence 5' CCA TTG TAR CAY GYG TGW AG 3', which is a particularly preferred sequence.

In one preferred embodiment a primer is used which consists of the sequence 5' CCA TTG TAR CAY GYG TGW AG 3'. As demonstrated in the examples, many members of Bifidobacteria, Bacteroides, Enterococci, Enterobacteria, Staphylococci, Lactobacilli, Lactococci and Escherichia are particularly well amplified using this preferred primer.

A common primer according to the present invention is preferably combined with a genus-specific primer. The resulting primer pair is capable of amplifying nucleic acid of interest, while at least in part avoiding non-specific amplification. One preferred embodiment provides a method according to the invention, further comprising providing said sample with a primer having a length of between 10 and 50 nucleotides, preferably having a length of between 10 and 30 nucleotides, capable of specifically amplifying nucleic acid of Bifidobacteria, Bacteroides, Enterococci, Enterobacteria, Staphylococci, Lactobacilli, Lactococci or Escherichia. With a combination of a common primer and the above mentioned genus-specific primer, nucleic acid of members of Bifidobacteria, Bacteroides, Enterococci, Enterobacteria, Staphylococci, Lactobacilli, Lactococci or Escherichia is preferably amplified. When use is made of at least two different genus-specific primers, for instance one primer capable of specifically annealing to Bifidobacteria sequences and a second primer capable of specifically annealing to Bacteroides sequences, only one kind of common primer according to the present invention is preferably used. Hence, only three different primers are needed for forming two primer pairs. Likewise, only four different primers are needed for forming three primer pairs. For instance, a common primer according to the invention together with a Bifidobacteria-specific primer, a Bacteroides-specific primer and an Enterococci-specific primer form three primer pairs capable of specifically amplifying Bifidobacteria sequences, Bacteroides sequences and Enterococci sequences.

A common primer according to the invention is preferably combined with at least three, preferably at least four, more preferably at least five, more preferably at least six, more preferably at least seven, most preferably at least eight different genus-specific primers capable of specifically amplifying nucleic acid of Bifidobacteria, Bacteroides, Enterococci, Enterobacteria, Staphylococci, Lactobacilli, Lactococci or Escherichia, which genus-specific primers are all capable of specifically binding a sequence which is less than 300 nucleotides located from the location where the common primer anneals. This way, the efficiency of amplification of nucleic acid of many species and/or strains of Bifidobacteria, Bacteroides, Enterococci, Enterobacteria, Staphylococci, Lactobacilli, Lactococci and/or Escherichia is optimal for quantitative amplification protocols, while non-specific nucleic acid amplification is at least in part avoided. Table 1 depicts preferred primers which are specific for Bifidobacteria, Bacteroides, Enterococci, Enterobacteria, Staphylococci, Lactobacilli, Lactococci and Escherichia. These primers are particularly well capable of annealing to multiple species and/or strains of the same genus, while amplification of other sequences is at least in part avoided. Moreover, even when combined with the same common primer according to the invention, each of these primers depicted in Table 1 is capable of specifically amplifying a genus-specific sequence with a length between 20 and 300 nucleotides, hence allowing efficient nucleic acid amplification. Of course, the sequence of the primers and probes according to the invention may vary to some extent without departing from the invention. The invention therefore provides a method for subjecting a sample to a nucleic acid amplification reaction wherein a sample is provided with a primer having a length of between 10 and 50 nucleotides, preferably having a length of between 10 and 30 nucleotides, comprising a sequence which is at least 80% homologous to at least part of a sequence as depicted in table 1, said part having at least 15 nucleotides. Preferably a primer is used which which is at least 85%, more preferably at least 90%, even more preferably at least 95%, most preferably at least 98% homologous to at least part of a sequence as depicted in Table 1, said part having at least 15 nucleotides. Preferably said part comprises at least 18 nucleotides. In one particularly preferred embodiment a primer is used which consists of a sequence as depicted in Table 1.

Said genus-specific primers are preferably combined with a common primer according to the invention. One embodiment thus provides a method for subjecting a sample to a nucleic acid amplification reaction comprising:
- providing said sample with a primer with a length of between 9 and 50 nucleotides, preferably with a length of between 9 and 40 nucleotides, more preferably with a length of between 15 and 25 nucleotides, which is capable of specifically binding to a 16S rRNA nucleic acid sequence corresponding to the nucleic acid sequence located from about nucleotide position 1226 to about 1242 in the 16S rRNA sequence of Escherichia coli K12 as depicted in Figure 1, said positions being indicated in Figure 1;
- providing said sample with a primer with a length of between 10 and 50 nucleotides comprising a sequence which is at least 80% homologous to at least part of a sequence as depicted in Table 1, said part having at least 15 nucleotides; and
- performing a nucleic acid amplification reaction. Said primer preferably has a length of between 10 and 30 nucleotides.

Preferably, said sample is provided with a common primer of the invention and a plurality of said genus-specific primers. Said sample is preferably provided with a common primer of the invention and at least two, preferably at least three, more preferably at least four, more preferably at least five, more preferably at least six, more preferably at least seven, most preferably at least eight different genus-specific primers, each genus-specific primer having a length of between 10 and 50 nucleotides, preferably having a length of between 10 and 30 nucleotides, and comprising a sequence which is at least 80% homologous to at least part of a sequence as depicted in Table 1, said part having at least 15 nucleotides. In a particularly preferred embodiment a sample is provided with a primer consisting of the sequence 5' CCA TTG TAR CAY GYG TGW AG 3' and with at least four, preferably at least six, preferably at least seven, most preferably eight primers depicted in Table 1.

A method according to the invention, comprising providing said sample with a primer consisting of a sequence which is at least 80% homologous to at least part of a sequence as depicted in Table 1, said part having at least 15 nucleotides, preferably at least 18 nucleotides, is therefore also provided. Said primer preferably consists of a sequence as depicted in Table 1.

One embodiment provides a method for screening for a genus-specific primer, comprising investigating whether a genus-specific nucleic acid sequence with a length of between 9 and 50 nucleotides, preferably with a length of between 9 and 40 nucleotides, more preferably with a length of between 15 and 25 nucleotides is present in a 16S rRNA sequence which is less than 300 nucleotides located from the location where a common primer according to the invention is capable of specifically binding. Of course, the sequence of a primer is preferably complementary to such genus-specific 16S rRNA sequence.

Amplified nucleic acid is preferably detected using a probe with a length of between 13 and 50 nucleotides, preferably between 13 and 30 nucleotides. Specific binding of a probe and, hence the presence of (amplified) nucleic acid having a sequence complementary to the sequence of the probe is for instance detected after the nucleic acid amplification reaction has taken place. Amplified nucleic acid is incubated with at least one probe, unbound probe is preferably washed away and bound probe is visualized, for instance using an enzyme reaction. Preferably however real time monitoring is performed, for instance using a Taqman or beacon probe. A Taqman probe comprises an oligonucleotide, complementary to a target sequence, which is labeled with a 5'-reporter dye and a 3'-quencher dye. When both dyes are attached to the probe the reporter dye cannot be detected. A TaqMan probe hybridizes to a target nucleic acid sequence. During a nucleic acid amplification reaction the TaqMan probe is hydrolyzed, resulting in separation of the reporter dye and quencher dyes. This, in turn, results in increased fluorescence of the reporter. Increase in fluorescence is monitored over time. Of course, many alternative methods for detection of amplified nucleic acid are available in the art.

In one embodiment at least one genus-specific probe is used in order to determine whether a certain genus is present in a sample. Preferably however a common probe is used which is capable of binding at least two, preferably at least four, more preferably at least six, most preferably at least eight different kinds of species and/or genera. Using a common probe further reduces the amount of nucleic acid sequences needed for amplification and detection of different species/genera. This is for instance illustrated by the following example. If four different species/genera are to be detected, current methods use four different species/genus-specific forward primers, four different species/genus-specific reverse primers and four different species/genus-specific probes. Hence, twelve different primer/probe sequences are needed in such current method. However, a method according to the present invention only needs one common primer, four different species/genus-specific primers and one common probe in order to detect four different species/genera. Hence, only six different primer/probe sequences are needed.

In one particularly preferred embodiment a probe is used with a length of between 13 and 50 nucleotides, preferably between 13 and 30 nucleotides, which comprises a sequence which is at least 80% homologous to at least part of the sequence 5' TCG TGY YRT SAR RTG TYR GST YA 3', said part having at least 18 nucleotides. This probe is capable of binding nucleic acid of Bifidobacteria, Bacteroides, Enterococci, Enterobacteria, Staphylococci, Lactobacilli, Lactococci and Escherichia and is a common probe according to the present invention.

Further provided is therefore a method according to the invention, further comprising detecting amplified nucleic acid with a probe with a length of between 13 and 50 nucleotides, preferably between 13 and 30 nucleotides, comprising a sequence which is at least 80% homologous to at least part of the sequence 5' TCG TGY YRT SAR RTG TYR GST YA 3', said part having at least 18 nucleotides, preferably at least 20 nucleotides. Said probe preferably comprises a sequence which is at least 85%, more preferably 90%, more preferably 95% and even more preferably at least 98% homologous to at least part of the sequence 5' TCG TGY YRT SAR RTG TYR GST YA 3', said part having at least 18 nucleotides, preferably at least 20 nucleotides. In one particularly preferred embodiment a probe is used which consists of the sequence 5' TCG TGY YRT SAR RTG TYR GST YA 3'.

A common primer according to the invention is capable of specifically hybridizing to 16S rRNA, which is present in prokaryotes. A common primer according to the invention is therefore particularly suitable for amplifying, detecting and/or isolating nucleic acid of prokaryotes. Further provided is therefore a method according to the invention wherein nucleic acid of prokaryotes is amplified. A method according to the present invention is preferably used for determining whether a sample comprises bacterial nucleic acid. Bacterial populations of complex systems, such as for instance biological samples (for instance derived from the gastrointestinal tract), environmental samples and/or starter cultures are preferably investigated using a method according to the present invention. Information about the kind and/or amount of various bacterial strains, species and/or genera in such complex systems is readily obtained using a method of the invention. Further provided is therefore a method for determining whether a sample comprises bacterial nucleic acid, comprising:
- subjecting said sample to a nucleic acid amplification reaction with a method according to the invention, and
- determining whether amplified nucleic acid is present.

It is preferably determined whether said sample comprises nucleic acid of at least one member of Bifidobacteria, Bacteroides, Enterococci, Enterobacteria, Staphylococci, Lactobacilli, Lactococci and/or Escherichia. A common primer according to the invention, as well as genus-specific primers according to the invention, are particularly suitable for annealing to at least one of these genera. In one preferred embodiment amplified nucleic acid is isolated and/or identified. This is for instance done with a species-specific and/or genus-specific probe. An array, (micro)chip and/or dipstick using complementary species-specific and/or genus-specific nucleic acid is suitable. Alternatively, or additionally, amplified nucleic acid is sequenced. Methods for isolating and/or identifying amplified nucleic acid are well known in the art and need no further explanation here. Non-limiting examples of identification methods are for instance outlined in Sambrook et al (1989).

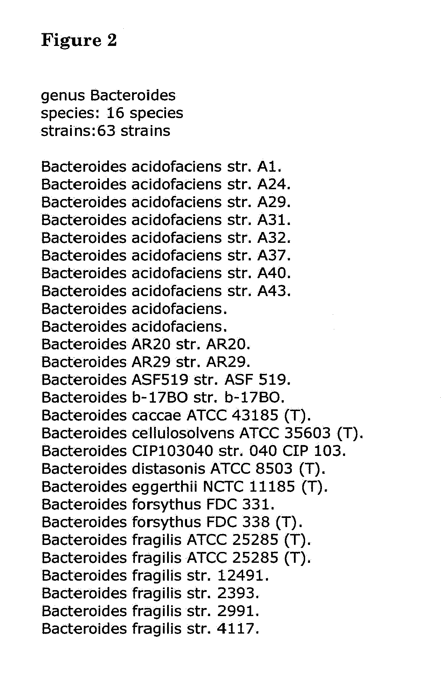

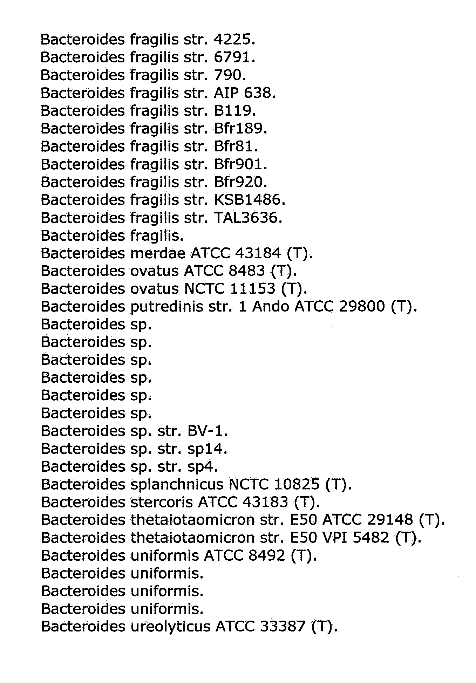

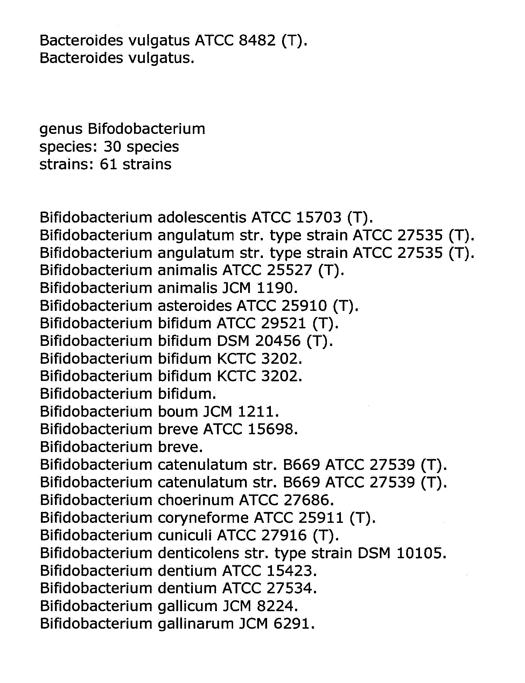

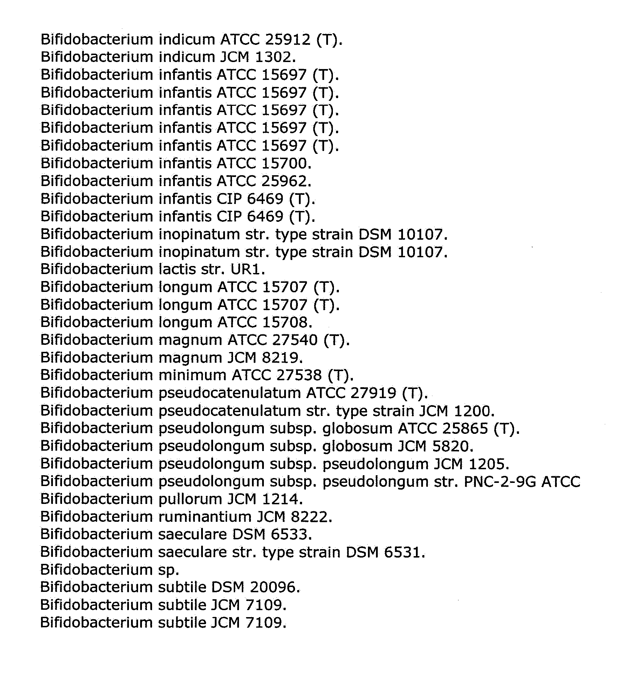

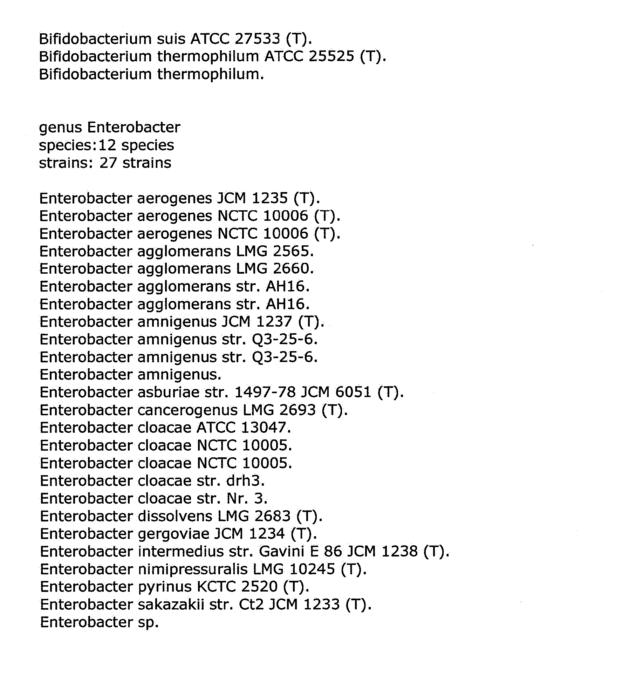

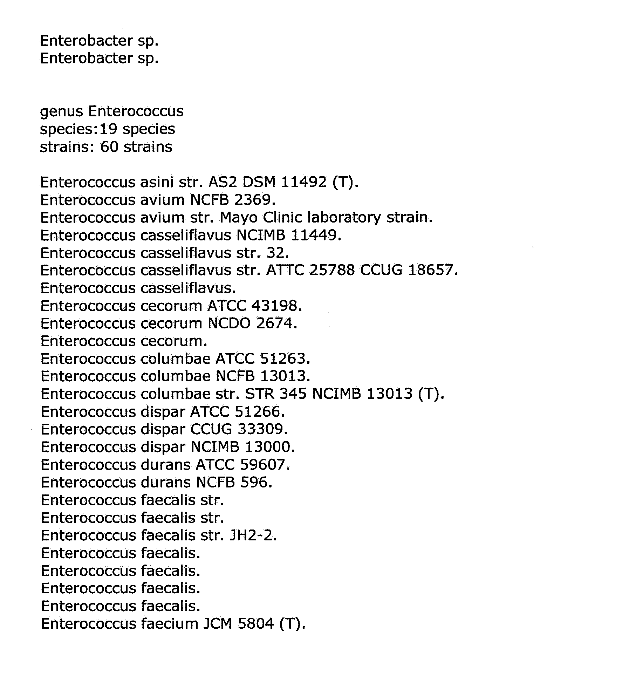

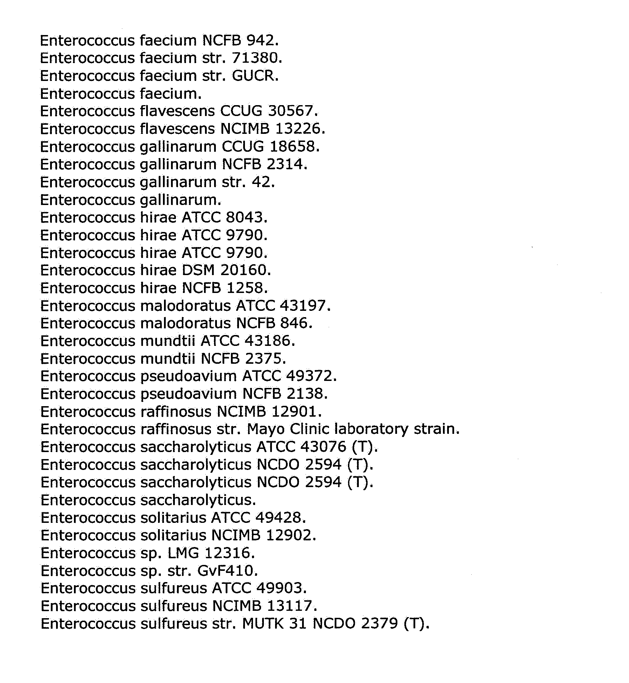

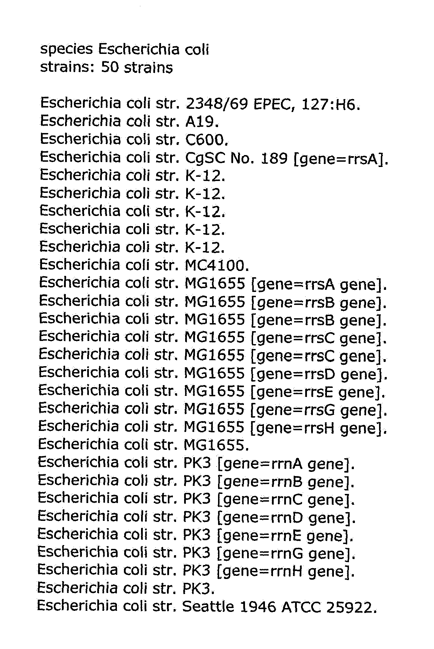

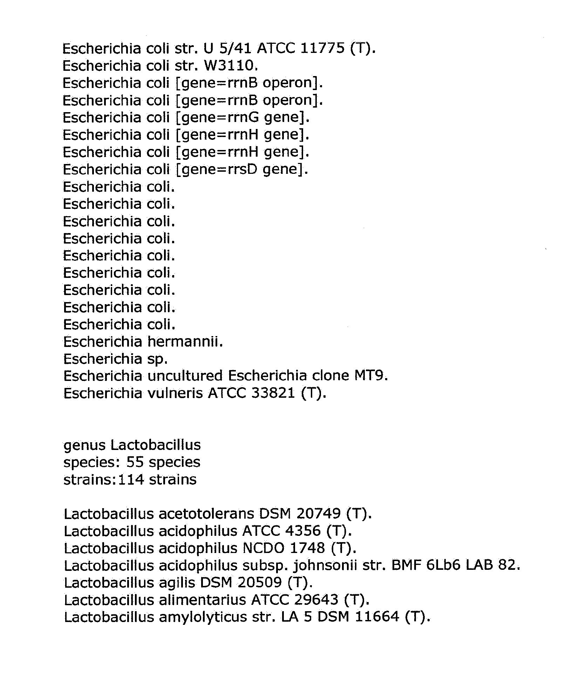

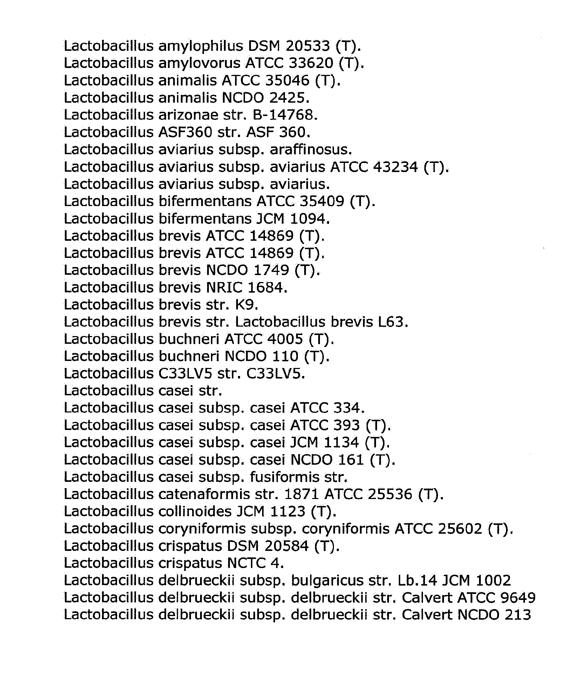

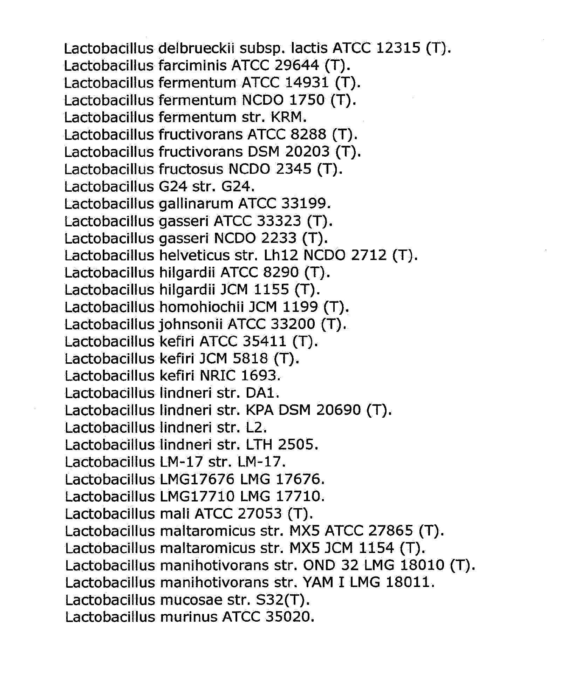

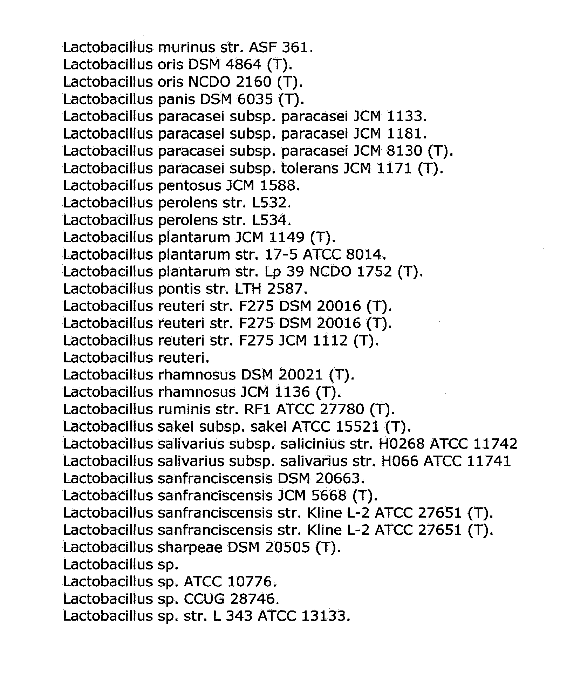

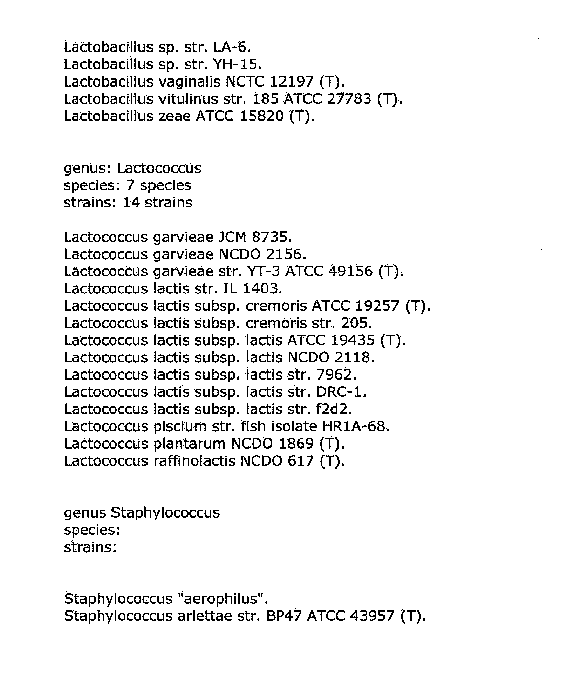

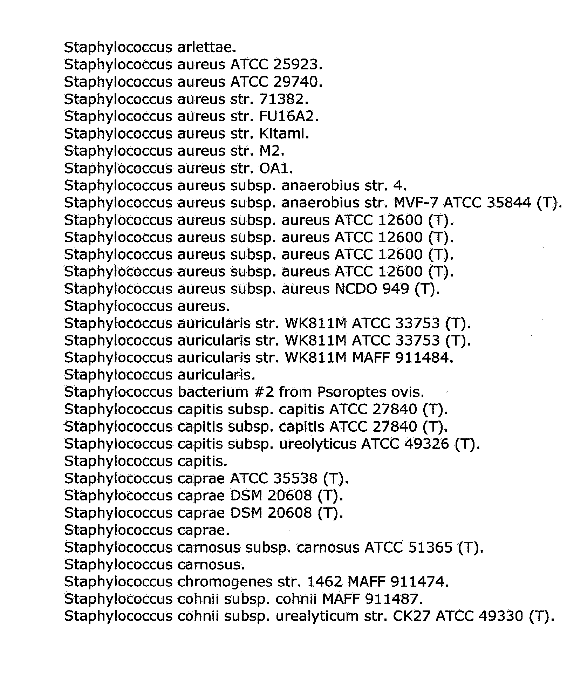

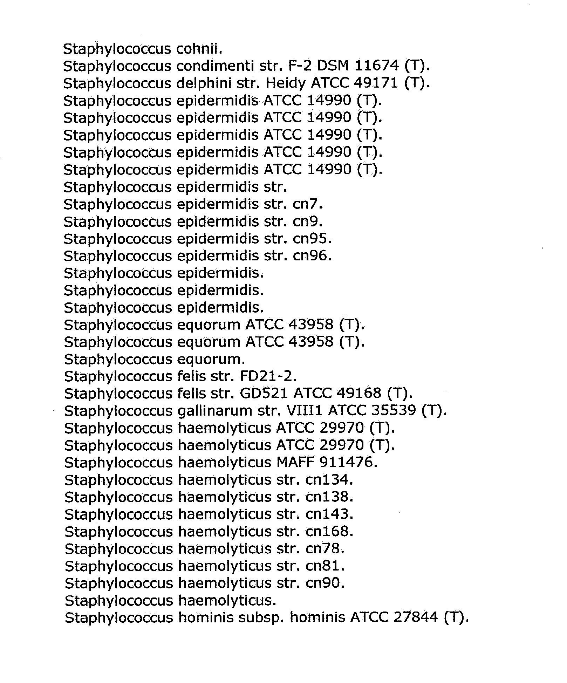

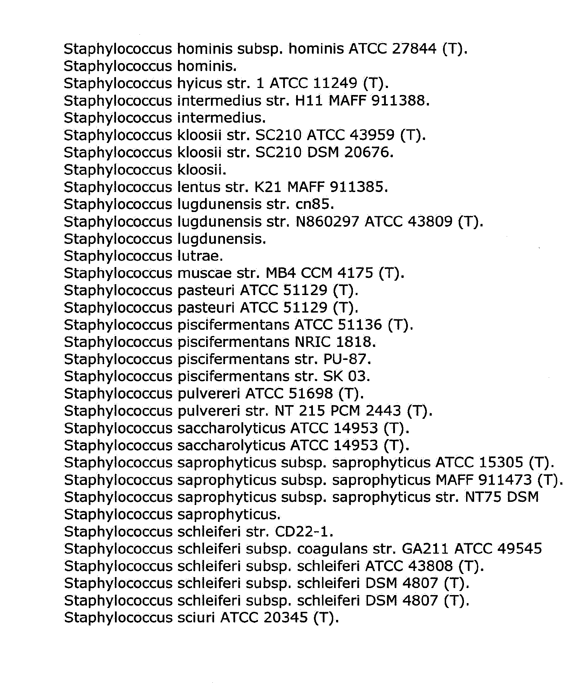

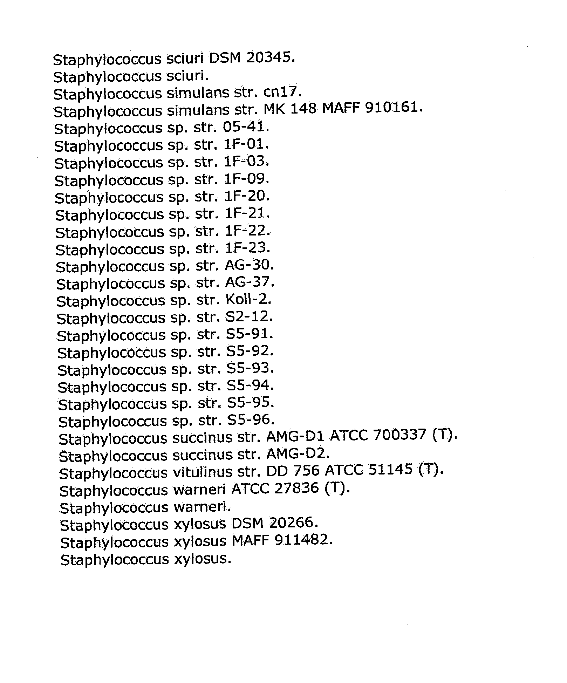

A method according to the invention is particularly suitable for amplifying a plurality of different nucleic acid sequences. Said method is preferably performed in order to test a sample for the presence of at least four different genera. More preferably, a sample is tested for the presence of at least five, preferably at least six, more preferably at least seven, more preferably at least eight different genera. The primers according to the present invention are capable of annealing to a wide variety of species and strains within a genus. Figure 2 depicts a non-limiting list of species and strains to which a common primer according to the invention is capable of specifically binding. A method according to the invention is therefore preferably performed in order to test a sample for the presence of at least one, preferably at least ten different bacterial species as depicted in Figure 2. A method according to the invention is furthermore preferably performed in order to test a sample for the presence of at least one, preferably at least ten different bacterial strains as depicted in Figure 2.

In yet another preferred embodiment amplified nucleic acid is quantified. Quantitation of nucleic acid is for instance performed for establishing whether a favourable equilibrium exists within various micro-organisms present in an environmental sample, a starter culture or a biological sample. Microbial nucleic acid present in a sample of the gastro-intestinal tract of an individual is for instance quantified because not only the kind of micro-organisms but also the relative amount of different species is indicative for such equilibrium. For instance, overgrowth of one kind of micro-organism, such as Clostridia species in the gastro-intestinal tract, is indicative for a less favourable health state or even disease. The composition of other populations, such as for instance the microbial populations of other biological samples (such as for instance a sample of the vaginal tract) or the microbial population of a starter culture or an environmental sample is also preferably investigated by quantifying specific species and/or genera. Preferably the relative amounts of several different species/genera relative to each other are determined. A method according to the invention, further comprising quantifying amplified nucleic acid, is therefore also provided.

Quantitation of nucleic acid is for instance performed using quantitative nucleic acid amplification, which is well known in the art. Known amounts of nucleic acid of interest are preferably amplified and visualised beforehand. The obtained values are plotted in order to obtain a reference curve. Subsequently, an unknown amount of nucleic acid is amplified and visualised, where after the obtained value is compared with said reference curve in order to establish the (original) amount of said nucleic acid of interest. In one embodiment real time quantitative nucleic acid amplification is used with Taqman or beacon probes. According to this embodiment an amplification plot is established in which a fluorescence signal is plotted versus the cycle number. In the initial cycles of a nucleic acid amplification reaction, there is generally little change in the fluorescence signal. This defines the baseline for the amplification plot. An increase in fluorescence above the baseline implies detection of accumulated nucleic acid amplification product. A fixed fluorescence threshold is preferably set above the baseline. According to this embodiment a parameter Ct is defined as the fractional cycle number at which the fluorescence passes the fixed threshold. Quantitation of the amount of target nucleic acid in unknown samples is subsequently preferably accomplished by measuring the Ct and using said standard curve to determine the starting copy number.
Of course, nucleic acid is also quantitated in various alternative ways, which are known in the art and need no further explanation.

A method according to the invention is, amongst other things, particularly suitable for investigating the presence and/or amount of a variety of bacterial species in the gastrointestinal tract. A faeces sample is particularly well suitable for this purpose. Further provided is therefore a method according to the invention for subjecting nucleic acid of a sample to a nucleic acid amplification reaction, wherein said nucleic acid is obtained from a faeces sample.

Further provided is a primer with a length of between 10 and 50 nucleotides, preferably with a length of between 10 and 30 nucleotides, capable of specifically binding to a 16S rRNA nucleic acid sequence corresponding to the nucleic acid sequence located from about nucleotide position 1226 to about 1242 in the 16S rRNA sequence of Escherichia coli K12 as depicted in Figure 1, said positions being indicated in Figure 1. As described before, such common primer is capable of annealing to a wide variety of bacterial genera. When combined with genus-specific primers, nucleic acid sequences with a length of between 20 and 300 nucleotides of many different genera is efficiently amplified. In a preferred aspect a primer of the invention is provided which is capable of specifically binding to said nucleic acid sequence under stringent conditions in order to diminish, or avoid, non-specific nucleic acid amplification. A common primer according to the invention preferably comprises a sequence which is at least 80%, more preferably at least 85%, more preferably at least 90%, more preferably at least 95%, most preferably at least 98% homologous to at least part of the sequence 5' CCA TTG TAR CAY GYG TGW AG 3', said part having at least 15 nucleotides, preferably at least 18 nucleotides. One embodiment provides a common primer which consists of the sequence 5' CCA TTG TAR CAY GYG TGW AG 3'.

The invention furthermore provides genus-specific primers. Primers comprising a sequence which is at least 80% homologous to at least part of the primer sequences depicted in Table 1 are preferred. Further provided is therefore a primer with a length of between 15 and 50 nucleotides, preferably with a length of between 10 and 30 nucleotides, capable of specifically amplifying nucleic acid of Bifidobacteria, Bacteroides, Enterococci, Enterobacteria, Staphylococci, Lactobacilli, Lactococci or Escherichia, said primer comprising a sequence which is at least 80%, more preferably at least 85%, more preferably at least 90%, more preferably at least 95%, most preferably at least 98%homologous to at least part of a sequence as depicted in table 1, said part having at least 15 nucleotides. Preferably said part comprises at least 18 nucleotides. A genus-specific primer consisting of a sequence as depicted in Table 1 is also herewith provided.

Furthermore provided is a probe with a length of between 13 and 50 nucleotides, preferably between 13 and 30 nucleotides, comprising a sequence which is at least 80%, more preferably at least 85%, more preferably at least 90%, more preferably at least 95%, most preferably at least 98% homologous to at least part of the sequence 5' TCG TGY YRT SAR RTG TYR GST YA 3', said part having at least 15 nucleotides, preferably at least 18 nucleotides. A probe consisting of the sequence 5' TCG TGY YRT SAR RTG TYR GST YA 3' is also herewith provided. As outlined before, a probe according to the invention is preferred since this is a common probe capable of binding, at least, nucleic acid of Bifidobacteria, Bacteroides, Enterococci, Enterobacteria, Staphylococci, Lactobacilli, Lactococci and Escherichia.

The invention furthermore provides a kit for detecting, identifying, isolating and/or quantifying bacterial nucleic acid, comprising a primer and/or a probe according to the invention. Said kit preferably comprises a common primer according to the invention in combination with at least one genus-specific probe, preferably a genus-specific primer comprising a sequence which is at least 80%, more preferably at least 85%, more preferably at least 90%, more preferably at least 95%, most preferably at least 98%homologous to at least part of a sequence as depicted in table 1, said part having at least 15 nucleotides, preferably at least 18 nucleotides. Most preferably a common primer according to the invention is combined with at least two, more preferably at least four, more preferably at least six, most preferably at least eight different genus-specific primers comprising a sequence which is at least 80%, more preferably at least 85%, more preferably at least 90%, more preferably at least 95%, most preferably at least 98%homologous to at least part of a sequence as depicted in Table 1, said part having at least 15 nucleotides.

A primer pair comprising a common primer according to the invention and a primer with a length of between 10 and 50 nucleotides, preferably with a length of between 10 and 30 nucleotides, capable of specifically amplifying nucleic acid of Bifidobacteria, Bacteroides, Enterococci, Enterobacteria, Staphylococci, Lactobacilli, Lactococci or Escherichia is also herewith provided. Said primer pair preferably comprises a common primer of the invention and a genus-specific primer which is capable of specifically binding a sequence which is at most 300 nucleotides located from a 16S rRNA nucleic acid sequence corresponding to the nucleic acid sequence located from about nucleotide position 1226 to about 1242 in the 16S rRNA sequence of Escherichia coli K12 as depicted in Figure 1, said positions being indicated in Figure 1. One embodiment provides a primer pair comprising a common primer according to the invention and a genus-specific primer comprising a sequence which is at least 80%, more preferably at least 85%, more preferably at least 90%, more preferably at least 95%, most preferably at least 98%homologous to at least part of a sequence as depicted in table 1, said part having at least 15 nucleotides. A particularly preferred primer pair comprises:
- a primer with a length of between 9 and 50 nucleotides, preferably with a length of between 9 and 40 nucleotides, more preferably with a length of between 15 and 25 nucleotides, which is capable of specifically binding to a 16S rRNA nucleic acid sequence corresponding to the nucleic acid sequence located from about nucleotide position 1226 to about 1242 in the 16S rRNA sequence of Escherichia coli K12 as depicted in Figure 1, said positions being indicated in Figure 1, and
- a primer consisting of a sequence as depicted in Table 1.

In one aspect a method according to the invention is used for testing whether a candidate compound is capable of influencing the amount of Bifidobacteria, Bacteroides, Enterococci, Enterobacteria, Staphylococci, Lactobacilli, Lactococci and/or Escherichia in a population. This is for instance useful for identifying, developing and/or testing new probiotics and/or prebiotics. Probiotics and prebiotics are useful for improving and/or restoring a normal healthy equilibrium in an individual's digestive tract. Probiotics are live micro-organisms, such as Bifidobacterium and/or Lactobacillus species, that are capable of improving the balance of the intestinal microflora. Probiotic bacterial cultures assist the body's naturally occurring flora within the digestive tract to reestablish themselves. Prebiotics are food ingredients that beneficially affect the host by selectively stimulating the growth and/or the activity of a limited number of bacteria in the colon. In order to monitor the effect of a certain probiotic or prebiotic, or in order to screen candidate compounds for beneficial activity, the effects upon a wide variety of micro-organisms is preferably assessed. This is preferably done by determining the presence and/or amount of a plurality of bacterial genera in a sample of an individual that has been provided with said (candidate) probiotic and/or (candidate) prebiotic, using a method according to the present invention. Preferably, a first sample of an individual is investigated with a method according to the invention before said individual has been provided with a (candidate) probiotic and/or (candidate) prebiotic. Subsequently, the same kind of sample (called herein a second sample) of said individual is investigated with a method according to the invention which second sample is obtained after said individual has been provided with a (candidate) probiotic and/or (candidate) prebiotic. Alterations in microbial composition of said first and second sample is indicative for an effect of said (candidate) probiotic and/or (candidate) prebiotic. Further provided is therefore a method for determining whether a (candidate) compound is capable of influencing the amount and/or concentration of Bifidobacteria, Bacteroides, Enterococci, Enterobacteria, Staphylococci, Lactobacilli, Lactococci and/or Escherichia in a population, comprising:
- providing said population with said (candidate) compound, and
- determining the presence and/or amount of nucleic acid of at least one member of Bifidobacteria, Bacteroides, Enterococci, Enterobacteria, Staphylococci, Lactobacilli, Lactococci and/or Escherichia in a sample of said population with a method according to the invention. The presence and/or amount of nucleic acid of at least one of said members in said sample is preferably compared with the presence and/or amount of nucleic acid of at least one of said members in a reference sample of said population, obtained before said population had been provided with said (candidate) compound.

Said population preferably comprises a population of the gastrointestinal tract. If a (candidate) compound appears to be capable of beneficially influencing the amount and/or concentration of Bifidobacteria, Bacteroides, Enterococci, Enterobacteria, Staphylococci, Lactobacilli, Lactococci and/or Escherichia in said population, it is preferably selected and/or isolated. A method according to the invention, further comprising selecting and/or isolating a candidate compound capable of influencing the amount and/or concentration of Bifidobacteria, Bacteroides, Enterococci, Enterobacteria, Staphylococci, Lactobacilli, Lactococci and/or Escherichia in said population is therefore also provided. Said selected and/or isolated compound is preferably incorporated into a food product, in order to improve health. Said food product preferably comprises a dairy product. A food product obtainable by a method according to the invention is therefore also provided.

The invention is further explained in the following examples. These examples do not limit the scope of the invention, but merely serve to clarify the invention.

### Examples

### Example 1

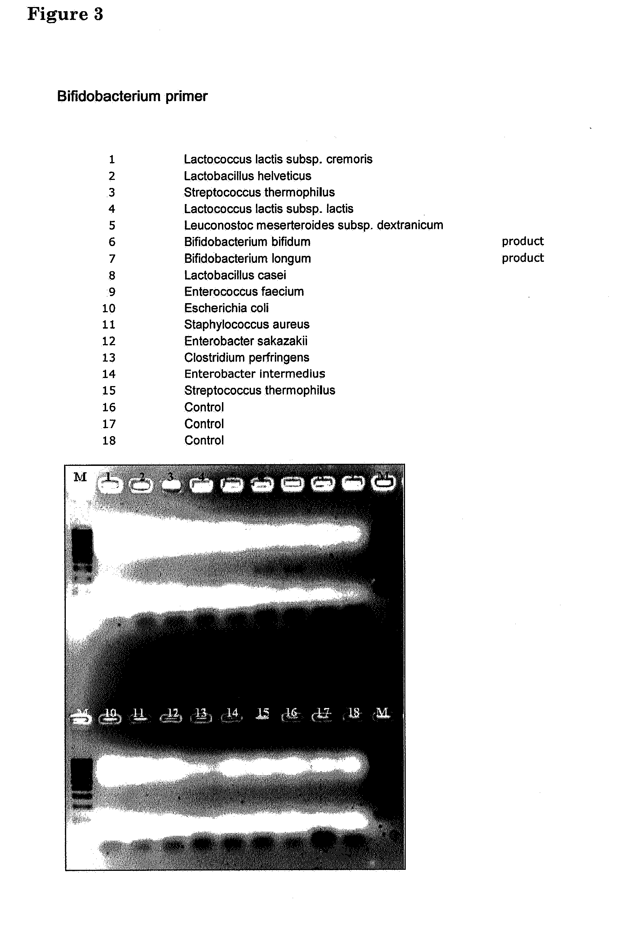

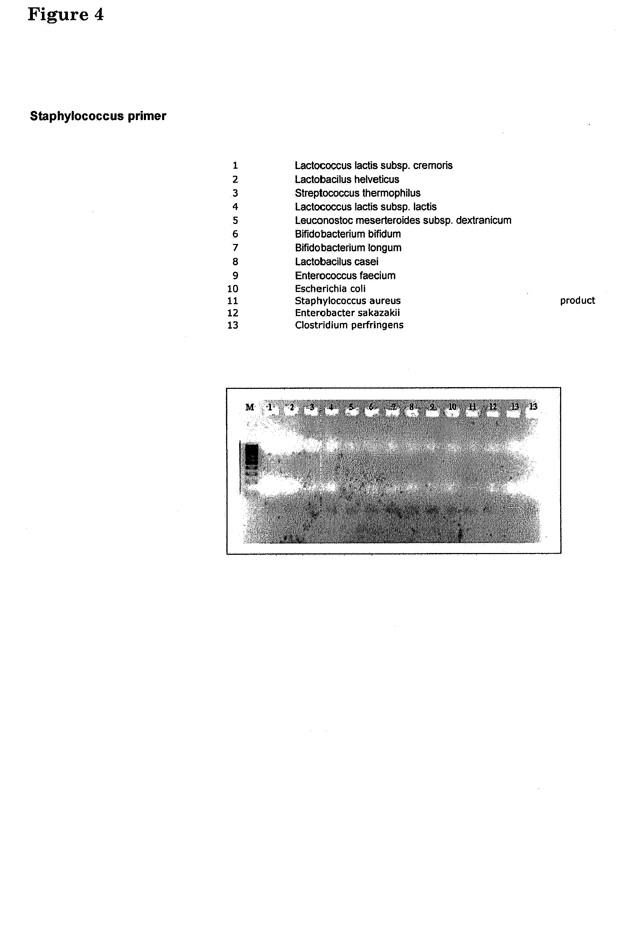

16s RNA DNA was amplified by PCR with oligonucleotide R20 Basic in combination with either F22 Bifidobacterium (Bifodobacterium) (Figure 3) or F21 Staphylococcus (Staphylococcus) (Figure 4).
oligonucleotide R20 Basic: CCA TTG TAR CAY GYG TGW AG.
R stands for a purine (G or A), Y stands for a pyrimidine (T or U or C) and W stands for an A or a T or a U.
F22 Bifidobacterium: ACC TTA CCT GGG CTT GAC ATG T
F21 Staphylococcus : CGC GAA GAA CCT TAC CAA ATC

### Reaction conditions:

- 425 ul Universeel Mastermix 25x cycle
- 85 ul R20Basic -> 40 pmol/ul Tm 62oC
- 85 ul F22Bifidoba
- 136 ul 25 mM
   MgCl2 4.0 mM MgCl2
- 34 ul demi
   - 5 ul template (~ 5 ng.ul)
- 280 ul Universeel Mastermix
- 70 ul forward F21
   primer Staphylococcus
- 280 ul reverse
   primer R20Basic
   - 5 ul template

### Programme:

- 7 min. 94oC
   repeat
   30x:
- 94oC 1 min
- 55oC 1 min.
- 72oC 2 min
   next
- 72oC 10 min
   hold
   10oC

The results are shown in Figures 3 and 4.

Figure 3 shows that, using oligonucleotide R20 Basic in combination with F22 Bifidobacterium, only Bifidobacterium nucleic acid is amplified. Both Bifidobacterium species are amplified.

Figure 4 shows that, using oligonucleotide R20 Basic in combination with F21 Staphylococcus, only Staphylococcus nucleic acid is amplified.

Hence, oligonucleotide R20 Basic is capable of amplifying nucleic acid of different kinds of micro-organisms, depending on the genus-specific primer with which oligonucleotide R20 Basic is combined. Thus, the general primer R20 Basic is capable of amplifying nucleic acid of various kinds of micro-organisms.

**Table 1 Genus-specific primers**

| | | |
|---|---|---|
| Bacteroides | 5' TTA CCC GGG CTT AAA TTG CA 3' | primer 1 |
| Bifidobacteria | 5' ACC TTA CCT GGG CTT GAC ATG T 3' | primer 2 |
| Enterobacteria | 5' AGA ACT TAG CAG AGA TGC TTT 3' | primer 3 |
| Enterococci | 5'ACC ACT CTA GAG ATA GAG CTT 3' | primer 4 |
| E. coli | 5' TCT TGA CAT CCA CGG AAG TT 3' | primer 5 |
| Lactobacilli | 5' CCT TAC CAG GTC TTG ACA TCT 3' | primer 6 |
| Lactococci | 5' CTATTCCTAGAGATAGGAAGTT 3' | primer 7 |
| Staphylococci | 5' CGC GAA GAA CCT TAC CAA ATC 3' | primer 8 |

### Brief description of the drawings

**Figure 1****.** Alignment of 16s rRNA sequences. The 16s rRNA sequence of E. coli K12 represents nucleotide position 223771 - 225312 of the complete genome. Entry: NC_000913 GeneID:944897 16S ribosomal RNA E.coli K12 (Blattner,F.R., Plunkett,G. III, Bloch,C.A., Perna,N.T., Burland,V., Riley,M., Collado-Vides,J., Glasner,J.D., Rode,C.K., Mayhew,G.F., Gregor,J., Davis,N.W., Kirkpatrick,H.A., Goeden,M.A., Rose,D.J., Mau,B. and Shao,Y. (1997). The complete genome sequence of Escherichia coli K-12. Science 277 (5331), 1453-1474).
**Figure 2****.** Non-limiting list of species and strains to which a common primer according to the present invention is capable of specifically binding.
**Figure 3****.** Result of Example 1
**Figure 4****.** Result of Example 1

### References

Sambrook, J., Fritsch, E.F., and Maniatis, T. 1989. Molecular cloning: a laboratory manual. Cold Spring Harbor laboratory Press, Cold Spring Harbor, N.Y.

## Claims

1. A method for subjecting a sample to a nucleic acid amplification reaction comprising providing said sample with at least one primer and performing a nucleic acid amplification reaction, wherein said sample is provided with a primer with a length of between 9 and 50 nucleotides, preferably with a length of between 9 and 40 nucleotides, more preferably with a length of between 15 and 25 nucleotides, which primer is capable of specifically binding to a 16S rRNA nucleic acid sequence corresponding to the nucleic acid sequence located from about nucleotide position 1226 to about 1242 in the 16S rDNA sequence of Escherichia coli K12 as depicted in Figure 1, said positions being indicated in figure 1.

2. A method according to claim 1, wherein said primer is capable of specifically binding to said nucleic acid sequence under stringent conditions.

3. A method according to claim 1 or 2, wherein said sample comprises nucleic acid of at least one member of Bifidobacteria, Bacteroides, Enterococci, Enterobacteria, Staphylococci, Lactobacilli, Lactococci and/or Escherichia.

4. A method according to any one of claims 1-3, comprising providing said sample with a primer comprising a sequence which is at least 80% homologous to at least part of the sequence 5' CCA TTG TAR CAY GYG TGW AG 3', said part having at least 15 nucleotides, preferably at least 18 nucleotides.

5. A method according to any one of claims 1-4, further comprising providing said sample with a primer with a length of between 9 and 50 nucleotides, preferably with a length of between 9 and 40 nucleotides, more preferably with a length of between 15 and 25 nucleotides, capable of specifically amplifying nucleic acid of Bifidobacteria, Bacteroides, Enterococci, Enterobacteria, Staphylococci, Lactobacilli, Lactococci or Escherichia.

6. A method according to claim 5, comprising providing said sample with a primer comprising a sequence which is at least 80% homologous to at least part of a sequence as depicted in table 1, said part having at least 15 nucleotides, preferably at least 18 nucleotides.

7. A method according to any one of claims 1-6, further comprising detecting amplified nucleic acid with a probe with a length of between 13 and 50 nucleotides, preferably between 13 and 30 nucleotides, comprising a sequence which is at least 80% homologous to at least part of the sequence 5' TCG TGY YRT SAR RTG TYR GST YA 3', said part having at least 18 nucleotides, preferably at least 20 nucleotides.

8. A method for determining whether a sample comprises bacterial nucleic acid, comprising:
- subjecting said sample to a nucleic acid amplification reaction with a method according to any one of claims 1-7, and
- determining whether amplified nucleic acid is present.

9. A method according to claim 8, comprising determining whether said sample comprises nucleic acid of at least one member of Bifidobacteria, Bacteroides, Enterococci, Enterobacteria, Staphylococci, Lactobacilli, Lactococci and/or Escherichia.

10. A method according to claim 8 or 9, further comprising identifying amplified nucleic acid.

11. A method according to claim 10, wherein said sample is tested for the presence of at least one, preferably at least ten different species and/or strains.

12. A method according to any one of claims 1-11, further comprising quantifying amplified nucleic acid.

13. A method according to any one of claims wherein said nucleic acid is obtained from a faeces sample.

14. A primer with a length of between 9 and 50 nucleotides, preferably with a length of between 9 and 40 nucleotides, more preferably with a length of between 15 and 25 nucleotides, capable of specifically binding to a 16S rRNA nucleic acid sequence corresponding to the nucleic acid sequence located from about nucleotide position 1226 to about 1242 in the 16S rRNA sequence of Escherichia coli K12 as depicted in Figure 1, said positions being indicated in figure 1.

15. A primer according to claim 14, which is capable of specifically binding to said nucleic acid sequence under stringent conditions.

16. A primer according to claim 14 or 15, comprising a sequence which is at least 80% homologous to at least part of the sequence 5' CCA TTG TAR CAY GYG TGW AG 3', said part having at least 15 nucleotides, preferably at least 18 nucleotides.

17. A primer with a length of between 9 and 50 nucleotides, preferably with a length of between 9 and 40 nucleotides, more preferably with a length of between 15 and 25 nucleotides, which is capable of specifically amplifying nucleic acid of Bifidobacteria, Bacteroides, Enterococci, Enterobacteria, Staphylococci, Lactobacilli, Lactococci or Escherichia, said primer comprising a sequence which is at least 80% homologous to at least part of a sequence as depicted in table 1, said part having at least 15 nucleotides, preferably at least 18 nucleotides.

18. A probe with a length of between 13 and 50 nucleotides, preferably between 13 and 30 nucleotides, comprising a sequence which is at least 80% homologous to at least part of the sequence 5' TCG TGY YRT SAR RTG TYR GST YA 3', said part having at least 15 nucleotides, preferably at least 18 nucleotides.

19. A kit for detecting, identifying and/or quantifying bacterial nucleic acid, comprising a primer according to any one of claims 14-17 and/or a probe according to claim 18.

20. A primer pair comprising a primer according to any one of claims 14-16 and a primer with a length of between 9 and 50 nucleotides, preferably with a length of between 9 and 40 nucleotides, more preferably with a length of between 15 and 25 nucleotides, capable of specifically amplifying nucleic acid of Bifidobacteria, Bacteroides, Enterococci, Enterobacteria, Staphylococci, Lactobacilli, Lactococci or Escherichia.

21. A primer pair according to claim 20, comprising a primer according to any one of claims 14-16 and a primer according to claim 17.

22. A method for determining whether a candidate compound is capable of influencing the amount of Bifidobacteria, Bacteroides, Enterococci, Enterobacteria, Staphylococci, Lactobacilli, Lactococci and/or Escherichia in a population, comprising:
- providing said population with said candidate compound, and
- determining the presence and/or amount of nucleic acid of at least one member of Bifidobacteria, Bacteroides, Enterococci, Enterobacteria, Staphylococci, Lactobacilli, Lactococci and/or Escherichia in a sample of said population with a method according to any one of claims 1-12.

23. A method according to claim 22, wherein said population comprises a population of the gastrointestinal tract.

24. A method according to claim 22 or 23, further comprising selecting and/or isolating a candidate compound capable of influencing the amount of Bifidobacteria, Bacteroides, Enterococci, Enterobacteria, Staphylococci, Lactobacilli, Lactococci and/or Escherichia in said population.

25. A method according to claim 24, further incorporating said selected and/or isolated compound into a food product.


SEQUENCE LISTING

<110> Friesland Brands B.V.

<120> Nucleic acid amplification

<130> P76906EP00

<140> EP 07109429.6
<141> 2007-06-01

<160> 20

<170> PatentIn version 3.3

<210> 1
<211> 1542
<212> DNA
<213> Artificial

<220>
<223> consensus


<220>
<221> misc_feature
<222> (71)..(72)
<223> n is a, c, g, or t

<220>
<221> misc_feature
<222> (76)..(76)
<223> n is a, c, g, or t

<220>
<221> misc_feature
<222> (85)..(85)
<223> n is a, c, g, or t

<220>
<221> misc_feature
<222> (87)..(89)
<223> n is a, c, g, or t

<220>
<221> misc_feature
<222> (184)..(184)
<223> n is a, c, g, or t

<220>
<221> misc_feature
<222> (186)..(186)
<223> n is a, c, g, or t

<220>
<221> misc_feature
<222> (189)..(189)
<223> n is a, c, g, or t

<220>
<221> misc_feature
<222> (199)..(202)
<223> n is a, c, g, or t

<220>
<221> misc_feature
<222> (476)..(476)
<223> n is a, c, g, or t

<220>
<221>  misc_feature
<222>  (1022)..(1023)
<223>  n is a, c, g, or t

<220>
<221>  misc_feature
<222>  (1038)..(1038)
<223>  n is a, c, g, or t

<220>
<221>  misc_feature
<222>  (1484)..(1486)
<223>  n is a, c, g, or t

<220>
<221>  misc_feature
<222>  (1490)..(1542)
<223>  n is a, c, g, or t

<400>  1
tggagagttt gatcctggct caggatgaac gctggcggcg tgcctaacac atgcaagtcg      60

aacggtaaca nnaagnagct tgctncnnng gtgacgggtg gcggacgggt gagtaacgcg     120

tgggaaacct gcctgttaga gggggataac tactggaaac ggttgctaat accgcataac     180

atcntnaanc gcatggtgnn nnacttaaag ggcttttgca ctatcggatg ggcccgcgtt     240

gtattagcta gttggtgggg taacggctca ccaaggcgac gatgcgtagc cggtctgaga     300

gggtgatcgg ccacactgga actgagacac ggtccagact cctacgggag gcagcagtgg     360

ggaatattgc acaatgggcg caagcctgat gcagccacgc cgcgtgagtg aagaaggcct     420

tcgggttgta aaactctttt attggggaag aagggggtga gagtaatatg ttcacncatt     480

gacggtacct aacgaaaaag caccggctaa ctacgtgcca gcagccgcgg taatacgtag     540

gttgcaagcg ttatccggat ttattgggcg taaagcgcgc gcaggcggtt tgttaagtct     600

gatgtgaaag cccccggctc aaccgtggaa gtgcattgga aactggcagg cttgagtgca     660

gtagaggaga gtggaattcc aggtgtagcg gtgaaatgcg tagatatctg gaggaacacc     720

ggtggcgaag gcggctctct ggactgtaac tgacgctgag gtgcgaaagc gtggggagca     780

aacaggatta gataccctgg tagtccacgc cgtaaacgat gagtgctagg tgtttgtggc     840

tttcagccgt tggtgccgga gctaacgcat taagcagtcc gcctggggag tacggccgca     900

aggttgaaac tcaaaggaat tgacgggggc ccgcacaagc ggtggagcat gtggtttaat     960

tcgatgcaac gcgaagaacc ttacctggtc ttgacatcct ctgaactttc tagagatagg    1020

gnnttccctt cggggacngt gtgacaggtg gtgcatggtt gtcgtcagct cgtgtcgtga    1080

gatgttgggt taagtcccgc aacgagcgca acccttatcc ttagttgcca gcagttcagt    1140

tgggcactct agggagactg ccggtgataa accggaggaa ggtggggatg acgtcaaatc    1200

atcatgcccc ttacgacctg ggctacacac gtgctacaat ggcgggtaca aagagaagcg    1260

acctcgcgag ggcaagcgaa tctcttaaag ccggtctcag ttcggattgg agtctgcaac    1320

tcgactccat gaagtcggaa tcgctagtaa tcgcggatca gcatgccgcg gtgaatacgt    1380

tcccgggcct tgtacacacc gcccgtcaca ccatgggagt tggtagcacc cgaagtcggt 1440

ggcttaacct tggggaggga gccgcctaag gtgggactga tgannngggn nnnnnnnnnn 1500

nnnnnnnnnn nnnnnnnnnn nnnnnnnnnn nnnnnnnnnn nn 1542

<210> 2
<211> 1542
<212> DNA
<213> Escherichia coli

<400> 2
aaattgaaga gtttgatcat ggctcagatt gaacgctggc ggcaggccta acacatgcaa 60

gtcgaacggt aacaggaaga agcttgcttc tttgctgacg agtggcggac gggtgagtaa 120

tgtctgggaa actgcctgat ggagggggat aactactgga aacggtagct aataccgcat 180

aacgtcgcaa gaccaaagag ggggaccttc gggcctcttg ccatcggatg tgcccagatg 240

ggattagcta gtaggtgggg taacggctca cctaggcgac gatccctagc tggtctgaga 300

ggatgaccag ccacactgga actgagacac ggtccagact cctacgggag gcagcagtgg 360

ggaatattgc acaatgggcg caagcctgat gcagccatgc cgcgtgtatg aagaaggcct 420

tcgggttgta aagtactttc agcggggagg aagggagtaa agttaatacc tttgctcatt 480

gacgttaccc gcagaagaag caccggctaa ctccgtgcca gcagccgcgg taatacggag 540

ggtgcaagcg ttaatcggaa ttactgggcg taaagcgcac gcaggcggtt tgttaagtca 600

gatgtgaaat ccccgggctc aacctgggaa ctgcatctga tactggcaag cttgagtctc 660

gtagaggggg gtagaattcc aggtgtagcg gtgaaatgcg tagagatctg gaggaatacc 720

ggtggcgaag gcggccccct ggacgaagac tgacgctcag gtgcgaaagc gtggggagca 780

aacaggatta gataccctgg tagtccacgc cgtaaacgat gtcgacttgg aggttgtgcc 840

cttgaggcgt ggcttccgga gctaacgcgt taagtcgacc gcctggggag tacggccgca 900

aggttaaaac tcaaatgaat tgacgggggc ccgcacaagc ggtggagcat gtggtttaat 960

tcgatgcaac gcgaagaacc ttacctggtc ttgacatcca cagaactttc cagagatgga 1020

ttggtgcctt cgggaactgt gagacaggtg ctgcatggct gtcgtcagct cgtgttgtga 1080

aatgttgggt taagtcccgc aacgagcgca acccttatct tttgttgcca gcggtccggc 1140

cgggaactca aaggagactg ccagtgataa actggaggaa ggtggggatg acgtcaagtc 1200

atcatggccc ttacgaccag ggctacacac gtgctacaat ggcgcataca aagagaagcg 1260

acctcgcgag agcaagcgga cctcataaag tgcgtcgtag tccggattgg agtctgcaac 1320

tcgactccat gaagtcggaa tcgctagtaa tcgtggatca gaatgccacg gtgaatacgt 1380

tcccgggcct tgtacacacc gcccgtcaca ccatgggagt gggttgcaaa agaagtaggt 1440

agcttaacct tcgggagggc gcttaccact ttgtgattca tgactggggt gaagtcgtaa 1500

caaggtaacc gtaggggaac ctgcggttgg atcacctcct ta 1542

<210> 3
<211> 1451

<212>  DNA
<213>  Artificial

<220>
<223>  Escherichia consensus 2


<220>
<221>  misc_feature
<222>  (1)..(5)
<223>  n is a, c, g, or t

<220>
<221>  misc_feature
<222>  (71)..(74)
<223>  n is a, c, g, or t

<220>
<221>  misc_feature
<222>  (80)..(82)
<223>  n is a, c, g, or t

<220>
<221>  misc_feature
<222>  (84)..(85)
<223>  n is a, c, g, or t

<220>
<221>  misc_feature
<222>  (184)..(185)
<223>  n is a, c, g, or t

<220>
<221>  misc_feature
<222>  (202)..(202)
<223>  n is a, c, g, or t

<220>
<221>  misc_feature
<222>  (304)..(304)
<223>  n is a, c, g, or t

<220>
<221>  misc_feature
<222>  (334)..(334)
<223>  n is a, c, g, or t

<220>
<221>  misc_feature
<222>  (393)..(393)
<223>  n is a, c, g, or t

<220>
<221>  misc_feature
<222>  (840)..(840)
<223>  n is a, c, g, or t

<220>
<221>  misc_feature
<222>  (853)..(854)
<223>  n is a, c, g, or t

<220>
<221>  misc_feature
<222>  (1019)..(1019)
<223>  n is a, c, g, or t

<220>

<221>  misc_feature
<222>  (1089)..(1089)
<223>  n is a, c, g, or t

<220>
<221>  misc_feature
<222>  (1373)..(1375)
<223>  n is a, c, g, or t

<220>
<221>  misc_feature
<222>  (1430)..(1432)
<223>  n is a, c, g, or t

<220>
<221>  misc_feature
<222>  (1440)..(1442)
<223>  n is a, c, g, or t

<400>  3
nnnnntgatc atggctcaga ttgaacgctg gcggcaggcc taacacatgc aagtcgaacg      60

gtaacaggaa nnnncttgcn nntnngctga cgagtggcgg acgggtgagt aatgtctggg     120

aaactgcctg atggaggggg ataactactg gaaacggtag ctaataccgc ataacgtcgc     180

aagnncaaag agggggacct tngggcctct tgccatcgga tgtgcccaga tgggattagc     240

tagtaggtgg ggtaacggct cacctaggcg acgatcccta gctggtctga gaggatgacc     300

agcnacactg gaactgagac acggtccaga ctcntacggg aggcagcagt ggggaatatt     360

gcacaatggg cgcaagcctg atgcagccat gcngcgtgta tgaagaaggc cttcgggttg     420

taaagtactt tcagcgggga ggaagggagt aaagttaata cctttgctca ttgacgttac     480

ccgcagaaga agcaccggct aactccgtgc cagcagccgc ggtaatacgg agggtgcaag     540

cgttaatcgg aattactggg cgtaaagcgc acgcaggcgg tttgttaagt cagatgtgaa     600

atccccgggc tcaacctggg aactgcatct gatactggca agcttgagtc tcgtagaggg     660

gggtagaatt ccaggtgtag cggtgaaatg cgtagagatc tggaggaata ccggtggcga     720

aggcggcccc ctggacgaag actgacgctc aggtgcgaaa gcgtggggag caaacaggat     780

tagataccct ggtagtccac gccgtaaacg atgtcgactt ggaggttgtg cccttgaggn     840

gtggcttccg ganntaacgc gttaagtcga ccgcctgggg agtacggccg caaggttaaa     900

actcaaatga attgacgggg gccgcacaag cggtggagca tgtggtttaa ttcgatgcaa     960

cgcgaagaac cttacctggt cttgacatcc acggaagttt tcagagatga gaatgtgcnt    1020

tcgggaaccg tgagacaggt gctgcatggc tgtcgtcagc tcgtgttgtg aaatgttggg    1080

ttaagtccng caacgagcgc aacccttatc ctttgttgcc agcggtccgg ccgggaactc    1140

aaaggagact gccagtgata aactggagga aggtggggat gacgtcaagt catcatggcc    1200

cttacgacca gggctacaca cgtgctacaa tggcgcatac aaagagaagc gacctcgcga    1260

gagcaagcgg acctcataaa gtgcgtcgta gtccggattg gagtctgcaa ctcgactcca    1320

tgaagtcgga atcgctagta atcgtggatc agaatgccac ggtgaatacg ttnnngggcc    1380

ttgtacacac cgcccgtcac accatgggag tgggttgcaa aagaagtagn nngcttaacn    1440

nngggagggc g                                                          1451

<210>  4
<211>  1483
<212>  DNA
<213>  Artificial

<220>
<223>  Bifidobacterium consensus 2


<220>
<221>  misc_feature
<222>  (1)..(2)
<223>  n is a, c, g, or t

<220>
<221>  misc_feature
<222>  (75)..(75)
<223>  n is a, c, g, or t

<220>
<221>  misc_feature
<222>  (77)..(84)
<223>  n is a, c, g, or t

<220>
<221>  misc_feature
<222>  (86)..(88)
<223>  n is a, c, g, or t

<220>
<221>  misc_feature
<222>  (90)..(94)
<223>  n is a, c, g, or t

<220>
<221>  misc_feature
<222>  (96)..(97)
<223>  n is a, c, g, or t

<220>
<221>  misc_feature
<222>  (130)..(130)
<223>  n is a, c, g, or t

<220>
<221>  misc_feature
<222>  (142)..(142)
<223>  n is a, c, g, or t

<220>
<221>  misc_feature
<222>  (144)..(144)
<223>  n is a, c, g, or t

<220>
<221>  misc_feature
<222>  (179)..(179)
<223>  n is a, c, g, or t

<220>
<221>  misc_feature
<222>  (183)..(183)
<223>  n is a, c, g, or t

<220>

<221>  misc_feature
<222>  (188)..(191)
<223>  n is a, c, g, or t

<220>
<221>  misc_feature
<222>  (193)..(193)
<223>  n is a, c, g, or t

<220>
<221>  misc_feature
<222>  (200)..(203)
<223>  n is a, c, g, or t

<220>
<221>  misc_feature
<222>  (205)..(205)
<223>  n is a, c, g, or t

<220>
<221>  misc_feature
<222>  (214)..(215)
<223>  n is a, c, g, or t

<220>
<221>  misc_feature
<222>  (219)..(220)
<223>  n is a, c, g, or t

<220>
<221>  misc_feature
<222>  (224)..(224)
<223>  n is a, c, g, or t

<220>
<221>  misc_feature
<222>  (239)..(239)
<223>  n is a, c, g, or t

<220>
<221>  misc_feature
<222>  (254)..(255)
<223>  n is a, c, g, or t

<220>
<221>  misc_feature
<222>  (258)..(258)
<223>  n is a, c, g, or t

<220>
<221>  misc_feature
<222>  (270)..(271)
<223>  n is a, c, g, or t

<220>
<221>  misc_feature
<222>  (275)..(276)
<223>  n is a, c, g, or t

<220>
<221>  misc_feature
<222>  (337)..(338)
<223>  n is a, c, g, or t

<220>
<221>  misc_feature
<222>  (364)..(365)
<223>  n is a, c, g, or t

<220>
<221>  misc_feature
<222>  (390)..(390)
<223>  n is a, c, g, or t

<220>
<221>  misc_feature
<222>  (403)..(403)
<223>  n is a, c, g, or t

<220>
<221>  misc_feature
<222>  (409)..(409)
<223>  n is a, c, g, or t

<220>
<221>  misc_feature
<222>  (437)..(437)
<223>  n is a, c, g, or t

<220>
<221>  misc_feature
<222>  (439)..(439)
<223>  n is a, c, g, or t

<220>
<221>  misc_feature
<222>  (443)..(443)
<223>  n is a, c, g, or t

<220>
<221>  misc_feature
<222>  (445)..(446)
<223>  n is a, c, g, or t

<220>
<221>  misc_feature
<222>  (456)..(467)
<223>  n is a, c, g, or t

<220>
<221>  misc_feature
<222>  (473)..(473)
<223>  n is a, c, g, or t

<220>
<221>  misc_feature
<222>  (478)..(479)
<223>  n is a, c, g, or t

<220>
<221>  misc_feature
<222>  (481)..(481)
<223>  n is a, c, g, or t

<220>
<221>  misc_feature
<222>  (534)..(534)
<223>  n is a, c, g, or t

<220>
<221>  misc_feature
<222>  (548)..(548)
<223>  n is a, c, g, or t

<220>
<221>  misc_feature

<222>  (620)..(620)
<223>  n is a, c, g, or t

<220>
<221>  misc_feature
<222>  (641)..(641)
<223>  n is a, c, g, or t

<220>
<221>  misc_feature
<222>  (733)..(733)
<223>  n is a, c, g, or t

<220>
<221>  misc_feature
<222>  (735)..(736)
<223>  n is a, c, g, or t

<220>
<221>  misc_feature
<222>  (753)..(753)
<223>  n is a, c, g, or t

<220>
<221>  misc_feature
<222>  (826)..(829)
<223>  n is a, c, g, or t

<220>
<221>  misc_feature
<222>  (922)..(922)
<223>  n is a, c, g, or t

<220>
<221>  misc_feature
<222>  (956)..(957)
<223>  n is a, c, g, or t

<220>
<221>  misc_feature
<222>  (998)..(999)
<223>  n is a, c, g, or t

<220>
<221>  misc_feature
<222>  (1001)..(1001)
<223>  n is a, c, g, or t

<220>
<221>  misc_feature
<222>  (1010)..(1010)
<223>  n is a, c, g, or t

<220>
<221>  misc_feature
<222>  (1012)..(1014)
<223>  n is a, c, g, or t

<220>
<221>  misc_feature
<222>  (1027)..(1027)
<223>  n is a, c, g, or t

<220>
<221>  misc_feature
<222>  (1041)..(1041)
<223>  n is a, c, g, or t

<220>
<221>  misc_feature
<222>  (1122)..(1124)
<223>  n is a, c, g, or t

<220>
<221>  misc_feature
<222>  (1126)..(1127)
<223>  n is a, c, g, or t

<220>
<221>  misc_feature
<222>  (1130)..(1131)
<223>  n is a, c, g, or t

<220>
<221>  misc_feature
<222>  (1145)..(1146)
<223>  n is a, c, g, or t

<220>
<221>  misc_feature
<222>  (1254)..(1254)
<223>  n is a, c, g, or t

<220>
<221>  misc_feature
<222>  (1261)..(1261)
<223>  n is a, c, g, or t

<220>
<221>  misc_feature
<222>  (1285)..(1285)
<223>  n is a, c, g, or t

<220>
<221>  misc_feature
<222>  (1300)..(1300)
<223>  n is a, c, g, or t

<220>
<221>  misc_feature
<222>  (1311)..(1311)
<223>  n is a, c, g, or t

<220>
<221>  misc_feature
<222>  (1317)..(1317)
<223>  n is a, c, g, or t

<220>
<221>  misc_feature
<222>  (1346)..(1346)
<223>  n is a, c, g, or t

<220>
<221>  misc_feature
<222>  (1357)..(1357)
<223>  n is a, c, g, or t

<220>
<221>  misc_feature
<222>  (1436)..(1436)
<223>  n is a, c, g, or t

<220>
<221>  misc_feature
<222>  (1442)..(1442)

<223>  n is a, c, g, or t

<220>
<221>  misc_feature
<222>  (1450)..(1451)
<223>  n is a, c, g, or t

<220>
<221>  misc_feature
<222>  (1472)..(1472)
<223>  n is a, c, g, or t

<220>
<221>  misc_feature
<222>  (1475)..(1483)
<223>  n is a, c, g, or t

<400>  4
nntttgtgga gggttcgatt ctggctcagg atgaacgctg gcggcgtgct taacacatgc      60

aagtcgaacg ggatncnnnn nnnntnnntn nnnngnngag agtggcgaac gggtgagtaa     120

tgcgtgaccn acctgcccca tncnccggaa tagctcctgg aaacgggtgg taatgccgna     180

tgntccannn nancgcatgn nnntntggga aagnntttnn cggnatggga tggggtcgng     240

tcctatcagc ttgnnggngg ggtaacggcn naccnnggct tcgacgggta gccggcctga     300

gagggcgacc ggccacattg ggactgagat acggccnnga ctcctacggg aggcagcagt     360

gggnnatatt gcacaatggg cgcaagcctn atgcagcgac gcngcgtgng ggatggaggc     420

cttcgggttg taaaccncnt ttntnnggga gcaagnnnnn nnnnnnntga gtntaccnnt     480

ngaataagca ccggctaact acgtgccagc agccgcggta atacgtaggg tgcnagcgtt     540

atccggantt attgggcgta aagggctcgt aggcggttcg tcgcgtccgg tgtgaaagtc     600

catcgcttaa cggtggatcn gcgccgggta cgggcgggct ngagtgcggt aggggagact     660

ggaattcccg gtgtaacggt ggaatgtgta gatatcggga agaacaccaa tggcgaaggc     720

aggtctctgg gcngnnactg acgctgagga gcnaaagcgt ggggagcgaa caggattaga     780

taccctggta gtccacgccg taaacggtgg atgctggatg tggggnnnnt tccacgggtt     840

ccgtgtcgga gctaacgcgt taagcatccc gcctggggag tacggccgca aggctaaaac     900

tcaaagaaat tgacgggggc cngcacaagc ggcggagcat gcggattaat tcgatnnaac     960

gcgaagaacc ttacctgggc ttgacatgtt cccgacgnnc ncagagatgn gnnntccctt    1020

cggggcnggt tcacaggtgg ngcatggtcg tcgtcagctc gtgtcgtgag atgttgggtt    1080

aagtcccgca acgagcgcaa ccctcgcccc gtgttgccag cnnntnntgn ngggaactca    1140

cgggnnaccg ccggggttaa ctcggaggaa ggtggggatg acgtcagatc atcatgcccc    1200

ttacgtccag ggcttcacgc atgctacaat ggccggtaca acgggatgcg acanggcgac    1260

ntggagcgga tccctgaaaa ccggnctcag ttcggatcgn agtctgcaac ncgactncgt    1320

gaaggcggag tcgctagtaa tcgcgnatca gcaacgncgc ggtgaatgcg ttcccgggcc    1380

ttgtacacac cgcccgtcaa gtcatgaaag tgggcagcac ccgaagccgg tggccnaacc    1440

cnttgtgggn nggagccgtc taaggtgagg cncgnnnnnn nnn                      1483

<210>  5
<211>  1448
<212>  DNA
<213>  Artificial

<220>
<223>  Enterobacterium consensus 2


<220>
<221>  misc_feature
<222>  (34)..(34)
<223>  n is a, c, g, or t

<220>
<221>  misc_feature
<222>  (58)..(58)
<223>  n is a, c, g, or t

<220>
<221>  misc_feature
<222>  (69)..(69)
<223>  n is a, c, g, or t

<220>
<221>  misc_feature
<222>  (426)..(426)
<223>  n is a, c, g, or t

<220>
<221>  misc_feature
<222>  (438)..(438)
<223>  n is a, c, g, or t

<220>
<221>  misc_feature
<222>  (442)..(442)
<223>  n is a, c, g, or t

<220>
<221>  misc_feature
<222>  (1102)..(1102)
<223>  n is a, c, g, or t

<400>  5
acgctggcgg caggcctaac acatgcaagt cgancggtag cacagagagc ttgctctngg      60

gtgacgagng gcggacgggt gagtaatgtc tgggaaactg cctgatggag ggggataact     120

actggaaacg gtagctaata ccgcataacg tcgcaagacc aaagaggggg accttcgggc     180

ctcttgccat cagatgtgcc cagatgggat tagctagtag gtggggtaac ggctcaccta     240

ggcgacgatc cctagctggt ctgagaggat gaccagccac actggaactg agacacggtc     300

cagactccta cgggaggcag cagtggggaa tattgcacaa tgggcgcaag cctgatgcag     360

ccatgccgcg tgtatgaaga aggccttcgg gttgtaaagt actttcagcg gggaggaagg     420

cgttgnggtt aataaccnca gngattgacg ttacccgcag aagaagcacc ggctaactcc     480

gtgccagcag ccgcggtaat acggagggtg caagcgttaa tcggaattac tgggcgtaaa     540

gcgcacgcag gcggtctgtc aagtcggatg tgaaatcccc gggctcaacc tgggaactgc     600

attcgaaact ggcaggctag agtcttgtag aggggggtag aattccaggt gtagcggtga     660

aatgcgtaga gatctggagg aataccggtg gcgaaggcgg ccccctggac aaagactgac 720

gctcaggtgc gaaagcgtgg ggagcaaaca ggattagata ccctggtagt ccacgccgta 780

aacgatgtcg acttggaggt tgtgcccttg aggcgtggct tccggagcta acgcgttaag 840

tcgaccgcct ggggagtacg gccgcaaggt taaaactcaa atgaattgac gggggcccgc 900

acaagcggtg gagcatgtgg tttaattcga tgcaacgcga agaaccttac ctactcttga 960

catccagaga acttagcaga gatgctttgg tgccttcggg aactctgaga caggtgctgc 1020

atggctgtcg tcagctcgtg ttgtgaaatg ttgggttaag tcccgcaacg agcgcaaccc 1080

ttatcctttg ttgccagcgg tncggccggg aactcaaagg agactgccag tgataaactg 1140

gaggaaggtg gggatgacgt caagtcatca tggcccttac gagtagggct acacacgtgc 1200

tacaatggcg catacaaaga gaagcgacct cgcgagagca agcggacctc ataaagtgcg 1260

tcgtagtccg gattggagtc tgcaactcga ctccatgaag tcggaatcgc tagtaatcgt 1320

agatcagaat gctacggtga atacgttccc gggccttgta cacaccgccc gtcacaccat 1380

gggagtgggt tgcaaaagaa gtaggtagct taaccttcgg gagggcgctt accactttgt 1440

gattcatg 1448

<210> 6
<211> 1562
<212> DNA
<213> Artificial

<220>
<223> Enterococcus consensus 2


<220>
<221> misc_feature
<222> (2)..(2)
<223> n is a, c, g, or t

<220>
<221> misc_feature
<222> (4)..(4)
<223> n is a, c, g, or t

<220>
<221> misc_feature
<222> (82)..(82)
<223> n is a, c, g, or t

<220>
<221> misc_feature
<222> (86)..(86)
<223> n is a, c, g, or t

<220>
<221> misc_feature
<222> (95)..(95)
<223> n is a, c, g, or t

<220>
<221> misc_feature
<222> (105)..(105)
<223> n is a, c, g, or t

<220>
<221> misc_feature
<222> (197)..(200)
<223> n is a, c, g, or t

<220>
<221> misc_feature
<222> (203)..(203)
<223> n is a, c, g, or t

<220>
<221> misc_feature
<222> (212)..(212)
<223> n is a, c, g, or t

<220>
<221> misc_feature
<222> (214)..(219)
<223> n is a, c, g, or t

<220>
<221> misc_feature
<222> (232)..(232)
<223> n is a, c, g, or t

<220>
<221> misc_feature
<222> (485)..(485)
<223> n is a, c, g, or t

<400> 6
cnsnststga gagtttgatc ctggctcagg acgaacgctg gcggcgtgcc taatacatgc 60

aagtcgaacg cttctttttt cnccgnagct tgctncamcg raaanagagg agtggcgaac 120

gggtgagtaa cacgtgggta acctgcccat cagmggggga taacacttgg aaacaggtgc 180

taataccgca taayaannnn wwnycgcatg rnannnnnnt gaaaggcgct tntgcgtcac 240

tgatggatgg acccgcggtg cattagctag ttggtgaggt aacggctyac caaggcmacg 300

atgcatagcc gacctgagag ggtgatcggc cacactggga ctgagacacg gcccagactc 360

ctacgggagg cagcagtagg gaatcttcgg caatggacga aagtctgacc gagcaacgcc 420

gcgtgagtga agaaggtttt cggatcgtaa aactctgttg ttagagaaga acaaggatga 480

gagknraatg ttcatccctt gacggtatct aaccagaaag ccacggctaa ctacgtgcca 540

gcagccgcgg taatacgtag gtggcaagcg ttgtccggat ttattgggcg taaagcgagc 600

gcaggcggtt tcttaagtct gatgtgaaag cccccggctc aaccggggag ggtcattgga 660

aactgggaga cttgagtgca gaagaggaga gtggaattcc atgtgtagcg gtgaaatgcg 720

tagatatatg gaggaacacc agtggcgaag gcggctctct ggtctgtaac tgacgctgag 780

gctcgaaagc gtggggagca aacaggatta gataccctgg tagtccacgc cgtaaacgat 840

gagtgctaag tgttggaggg tttccgccct tcagtgctgc agcwaacgca ttaagcactc 900

cgcctgggga gtacgaccgc aaggttgaaa ctcaaaggaa ttgacggggg cccgcacaag 960

cggtggagca tgtggtttaa ttcgaagcaa cgcgaagaac cttaccaggt cttgacatcc 1020

tttgaccact ctagagatag agctttccct tcggggacaa agtgacaggt ggtgcatggt 1080

tgtcgtcagc tcgtgtcgtg agatgttggg ttaagtcccg caacgagcgc aacccttatt 1140

gttagttgcc atcatttagt tgggcactct agcgagactg ccggtgacaa accggaggaa   1200

ggtggggatg acgtcaaatc atcatgcccc ttatgacctg ggctacacac gtgctacaat   1260

gggaagtaca acgagttgcg aagtcgcgag gctaagctaa tctcttaaag cttctctcag   1320

ttcggattgy aggctgcaac tcgcctrcat gaagccggaa tcgctagtaa tcgcggatca   1380

gcacgccgcg gtgaatacgt tcccgggcct tgtacacacc gcccgtcaca ccacgagagt   1440

ttgtaacacc cgaagtcggt gaggtaacct tttggagcca gccgcctaag gtgggataga   1500

tgatttgggt gaagtcgtaa caaggtaacc gtatcggaag gtgcggctgg atcacctcct   1560

ta                                                                  1562

<210>  7
<211>  1473
<212>  DNA
<213>  Artificial

<220>
<223>  Bacteroides consensus 2


<220>
<221>  misc_feature
<222>  (3)..(3)
<223>  n is a, c, g, or t

<220>
<221>  misc_feature
<222>  (75)..(75)
<223>  n is a, c, g, or t

<220>
<221>  misc_feature
<222>  (91)..(96)
<223>  n is a, c, g, or t

<220>
<221>  misc_feature
<222>  (141)..(142)
<223>  n is a, c, g, or t

<220>
<221>  misc_feature
<222>  (144)..(144)
<223>  n is a, c, g, or t

<220>
<221>  misc_feature
<222>  (147)..(147)
<223>  n is a, c, g, or t

<220>
<221>  misc_feature
<222>  (168)..(168)
<223>  n is a, c, g, or t

<220>
<221>  misc_feature
<222>  (181)..(182)
<223>  n is a, c, g, or t

<220>

<221>  misc_feature
<222>  (187)..(187)
<223>  n is a, c, g, or t

<220>
<221>  misc_feature
<222>  (191)..(191)
<223>  n is a, c, g, or t

<220>
<221>  misc_feature
<222>  (193)..(194)
<223>  n is a, c, g, or t

<220>
<221>  misc_feature
<222>  (196)..(196)
<223>  n is a, c, g, or t

<220>
<221>  misc_feature
<222>  (205)..(209)
<223>  n is a, c, g, or t

<220>
<221>  misc_feature
<222>  (220)..(220)
<223>  n is a, c, g, or t

<220>
<221>  misc_feature
<222>  (223)..(223)
<223>  n is a, c, g, or t

<220>
<221>  misc_feature
<222>  (229)..(229)
<223>  n is a, c, g, or t

<220>
<221>  misc_feature
<222>  (231)..(232)
<223>  n is a, c, g, or t

<220>
<221>  misc_feature
<222>  (254)..(254)
<223>  n is a, c, g, or t

<220>
<221>  misc_feature
<222>  (262)..(262)
<223>  n is a, c, g, or t

<220>
<221>  misc_feature
<222>  (282)..(282)
<223>  n is a, c, g, or t

<220>
<221>  misc_feature
<222>  (384)..(384)
<223>  n is a, c, g, or t

<220>
<221>  misc_feature
<222>  (388)..(388)
<223>  n is a, c, g, or t

<220>
<221>  misc_feature
<222>  (391)..(391)
<223>  n is a, c, g, or t

<220>
<221>  misc_feature
<222>  (407)..(407)
<223>  n is a, c, g, or t

<220>
<221>  misc_feature
<222>  (421)..(422)
<223>  n is a, c, g, or t

<220>
<221>  misc_feature
<222>  (425)..(425)
<223>  n is a, c, g, or t

<220>
<221>  misc_feature
<222>  (451)..(451)
<223>  n is a, c, g, or t

<220>
<221>  misc_feature
<222>  (464)..(467)
<223>  n is a, c, g, or t

<220>
<221>  misc_feature
<222>  (474)..(477)
<223>  n is a, c, g, or t

<220>
<221>  misc_feature
<222>  (491)..(491)
<223>  n is a, c, g, or t

<220>
<221>  misc_feature
<222>  (587)..(587)
<223>  n is a, c, g, or t

<220>
<221>  misc_feature
<222>  (590)..(591)
<223>  n is a, c, g, or t

<220>
<221>  misc_feature
<222>  (593)..(593)
<223>  n is a, c, g, or t

<220>
<221>  misc_feature
<222>  (648)..(649)
<223>  n is a, c, g, or t

<220>
<221>  misc_feature
<222>  (742)..(742)
<223>  n is a, c, g, or t

<220>
<221>  misc_feature

<222>  (756)..(756)
<223>  n is a, c, g, or t

<220>
<221>  misc_feature
<222>  (999)..(1001)
<223>  n is a, c, g, or t

<220>
<221>  misc_feature
<222>  (1006)..(1009)
<223>  n is a, c, g, or t

<220>
<221>  misc_feature
<222>  (1018)..(1022)
<223>  n is a, c, g, or t

<220>
<221>  misc_feature
<222>  (1024)..(1025)
<223>  n is a, c, g, or t

<220>
<221>  misc_feature
<222>  (1027)..(1029)
<223>  n is a, c, g, or t

<220>
<221>  misc_feature
<222>  (1033)..(1033)
<223>  n is a, c, g, or t

<220>
<221>  misc_feature
<222>  (1035)..(1037)
<223>  n is a, c, g, or t

<220>
<221>  misc_feature
<222>  (1117)..(1117)
<223>  n is a, c, g, or t

<220>
<221>  misc_feature
<222>  (1134)..(1134)
<223>  n is a, c, g, or t

<220>
<221>  misc_feature
<222>  (1151)..(1151)
<223>  n is a, c, g, or t

<220>
<221>  misc_feature
<222>  (1262)..(1262)
<223>  n is a, c, g, or t

<220>
<221>  misc_feature
<222>  (1265)..(1265)
<223>  n is a, c, g, or t

<220>
<221>  misc_feature
<222>  (1269)..(1269)
<223>  n is a, c, g, or t

<220>
<221>  misc_feature
<222>  (1276)..(1276)
<223>  n is a, c, g, or t

<220>
<221>  misc_feature
<222>  (1283)..(1283)
<223>  n is a, c, g, or t

<220>
<221>  misc_feature
<222>  (1287)..(1287)
<223>  n is a, c, g, or t

<220>
<221>  misc_feature
<222>  (1305)..(1305)
<223>  n is a, c, g, or t

<220>
<221>  misc_feature
<222>  (1307)..(1307)
<223>  n is a, c, g, or t

<220>
<221>  misc_feature
<222>  (1324)..(1324)
<223>  n is a, c, g, or t

<220>
<221>  misc_feature
<222>  (1326)..(1326)
<223>  n is a, c, g, or t

<220>
<221>  misc_feature
<222>  (1414)..(1415)
<223>  n is a, c, g, or t

<220>
<221>  misc_feature
<222>  (1435)..(1435)
<223>  n is a, c, g, or t

<220>
<221>  misc_feature
<222>  (1450)..(1450)
<223>  n is a, c, g, or t

<220>
<221>  misc_feature
<222>  (1453)..(1453)
<223>  n is a, c, g, or t

<400>  7
wcnatgaaga gtttgatcct ggctcaggat gaacgctagc tacaggctta acacatgcaa      60

gtcgaggggc agcanttyag tttgcttgca nnnnnngmtg gcgaccggcg cacgggtgag     120

taacacgtat ccaacctgcc nntnacncgg ggatagcctt tcgaaagnaa gattaatacc     180

nnatagnata ntnntnccgc atggnnnnnt tattaaagan ttntcggtna nngatgggga     240

tgcgttccat tagntagttg gnggggtaac ggcccaccaa gnctacgatg gataggggtt     300

ctgagaggaa ggtcccccac attggaactg agacacggtc caaactccta cgggaggcag     360

cagtgaggaa tattggtcaa tggncganag nctgaaccag ccaagtngcg tgaaggatga 420

nngcnctatg ggttgtaaac ttcttttata ngggaataaa gtgnnnnacg tgtnnnnttt 480

tgtatgtacc ntatgaataa ggatcggcta actccgtgcc agcagccgcg gtaatacgga 540

ggatccgagc gttatccgga tttattgggt ttaaagggag cgtaggnggn ntnttaagtc 600

agttgtgaaa gtttgcggct caaccgtaaa attgcagttg atactggnng tcttgagtac 660

agtagaggta ggcggaattc gtggtgtagc ggtgaaatgc ttagatatca cgaagaactc 720

cgattgcgaa ggcagcttac tngactgtaa ctgacnctga tgctcgaaag tgtgggtatc 780

aaacaggatt agataccctg gtagtccaca cagtaaacga tgaatactcg ctgtttgcga 840

tatacagtaa gcggccaagc gaaagcatta agtattccac ctggggagta cgccggcaac 900

ggtgaaactc aaaggaattg acgggggccc gcacaagcgg aggaacatgt ggtttaattc 960

gatgatacgc gaggaacctt acccgggctt aaattgcann ngaatnnnnt ggaaacannn 1020

nngnncnnna ggncnnntgt gaaggtgctg catggttgtc gtcagctcgt gccgtgaggt 1080

gtcggcttaa gtgccataac gagcgcaacc cttatcntta gttactaaca ggtnatgctg 1140

aggactctag ngagactgcc gtcgtaagat gtgaggaagg tggggatgac gtcaaatcag 1200

cacggccctt acgtccgggg ctacacacgt gttacaatgg ggggtacaga aggcagctac 1260

cnggngacng gatgcnaatc ccnaaancct ctctcagttc ggatngnagt ctgcaacccg 1320

actncntgaa gctggattcg ctagtaatcg cgcatcagcc atggcgcggt gaatacgttc 1380

ccgggccttg tacacaccgc ccgtcaagcc atgnnagccg ggggtacctg aagtncgtaa 1440

ccgcaaggan cgncctaggg taaaactggt ray 1473

<210> 8
<211> 1524
<212> DNA
<213> Artificial

<220>
<223> Lactobacillus consensus 2


<220>
<221> misc_feature
<222> (1)..(8)
<223> n is a, c, g, or t

<220>
<221> misc_feature
<222> (14)..(15)
<223> n is a, c, g, or t

<220>
<221> misc_feature
<222> (19)..(20)
<223> n is a, c, g, or t

<220>
<221> misc_feature
<222> (31)..(31)
<223> n is a, c, g, or t

<220>
<221>  misc_feature
<222>  (70)..(92)
<223>  n is a, c, g, or t

<220>
<221>  misc_feature
<222>  (99)..(122)
<223>  n is a, c, g, or t

<220>
<221>  misc_feature
<222>  (125)..(125)
<223>  n is a, c, g, or t

<220>
<221>  misc_feature
<222>  (151)..(151)
<223>  n is a, c, g, or t

<220>
<221>  misc_feature
<222>  (161)..(162)
<223>  n is a, c, g, or t

<220>
<221>  misc_feature
<222>  (166)..(167)
<223>  n is a, c, g, or t

<220>
<221>  misc_feature
<222>  (178)..(178)
<223>  n is a, c, g, or t

<220>
<221>  misc_feature
<222>  (189)..(189)
<223>  n is a, c, g, or t

<220>
<221>  misc_feature
<222>  (201)..(201)
<223>  n is a, c, g, or t

<220>
<221>  misc_feature
<222>  (207)..(207)
<223>  n is a, c, g, or t

<220>
<221>  misc_feature
<222>  (210)..(213)
<223>  n is a, c, g, or t

<220>
<221>  misc_feature
<222>  (215)..(215)
<223>  n is a, c, g, or t

<220>
<221>  misc_feature
<222>  (222)..(222)
<223>  n is a, c, g, or t

<220>
<221>  misc_feature
<222>  (224)..(230)

<223>  n is a, c, g, or t

<220>
<221>  misc_feature
<222>  (232)..(232)
<223>  n is a, c, g, or t

<220>
<221>  misc_feature
<222>  (237)..(237)
<223>  n is a, c, g, or t

<220>
<221>  misc_feature
<222>  (242)..(242)
<223>  n is a, c, g, or t

<220>
<221>  misc_feature
<222>  (244)..(245)
<223>  n is a, c, g, or t

<220>
<221>  misc_feature
<222>  (252)..(252)
<223>  n is a, c, g, or t

<220>
<221>  misc_feature
<222>  (255)..(257)
<223>  n is a, c, g, or t

<220>
<221>  misc_feature
<222>  (274)..(274)
<223>  n is a, c, g, or t

<220>
<221>  misc_feature
<222>  (296)..(296)
<223>  n is a, c, g, or t

<220>
<221>  misc_feature
<222>  (300)..(301)
<223>  n is a, c, g, or t

<220>
<221>  misc_feature
<222>  (310)..(310)
<223>  n is a, c, g, or t

<220>
<221>  misc_feature
<222>  (316)..(316)
<223>  n is a, c, g, or t

<220>
<221>  misc_feature
<222>  (326)..(327)
<223>  n is a, c, g, or t

<220>
<221>  misc_feature
<222>  (334)..(335)
<223>  n is a, c, g, or t

<220>

<221>  misc_feature
<222>  (337)..(337)
<223>  n is a, c, g, or t

<220>
<221>  misc_feature
<222>  (348)..(348)
<223>  n is a, c, g, or t

<220>
<221>  misc_feature
<222>  (367)..(369)
<223>  n is a, c, g, or t

<220>
<221>  misc_feature
<222>  (413)..(413)
<223>  n is a, c, g, or t

<220>
<221>  misc_feature
<222>  (417)..(417)
<223>  n is a, c, g, or t

<220>
<221>  misc_feature
<222>  (450)..(450)
<223>  n is a, c, g, or t

<220>
<221>  misc_feature
<222>  (457)..(457)
<223>  n is a, c, g, or t

<220>
<221>  misc_feature
<222>  (476)..(476)
<223>  n is a, c, g, or t

<220>
<221>  misc_feature
<222>  (485)..(489)
<223>  n is a, c, g, or t

<220>
<221>  misc_feature
<222>  (491)..(491)
<223>  n is a, c, g, or t

<220>
<221>  misc_feature
<222>  (503)..(503)
<223>  n is a, c, g, or t

<220>
<221>  misc_feature
<222>  (506)..(511)
<223>  n is a, c, g, or t

<220>
<221>  misc_feature
<222>  (521)..(522)
<223>  n is a, c, g, or t

<220>
<221>  misc_feature
<222>  (533)..(533)
<223>  n is a, c, g, or t

<220>
<221>  misc_feature
<222>  (578)..(578)
<223>  n is a, c, g, or t

<220>
<221>  misc_feature
<222>  (585)..(585)
<223>  n is a, c, g, or t

<220>
<221>  misc_feature
<222>  (622)..(625)
<223>  n is a, c, g, or t

<220>
<221>  misc_feature
<222>  (645)..(646)
<223>  n is a, c, g, or t

<220>
<221>  misc_feature
<222>  (652)..(652)
<223>  n is a, c, g, or t

<220>
<221>  misc_feature
<222>  (658)..(659)
<223>  n is a, c, g, or t

<220>
<221>  misc_feature
<222>  (679)..(681)
<223>  n is a, c, g, or t

<220>
<221>  misc_feature
<222>  (701)..(701)
<223>  n is a, c, g, or t

<220>
<221>  misc_feature
<222>  (726)..(726)
<223>  n is a, c, g, or t

<220>
<221>  misc_feature
<222>  (769)..(769)
<223>  n is a, c, g, or t

<220>
<221>  misc_feature
<222>  (779)..(779)
<223>  n is a, c, g, or t

<220>
<221>  misc_feature
<222>  (812)..(812)
<223>  n is a, c, g, or t

<220>
<221>  misc_feature
<222>  (855)..(855)
<223>  n is a, c, g, or t

<220>
<221>  misc_feature

<222>  (861)..(861)
<223>  n is a, c, g, or t

<220>
<221>  misc_feature
<222>  (890)..(890)
<223>  n is a, c, g, or t

<220>
<221>  misc_feature
<222>  (910)..(910)
<223>  n is a, c, g, or t

<220>
<221>  misc_feature
<222>  (965)..(965)
<223>  n is a, c, g, or t

<220>
<221>  misc_feature
<222>  (1000)..(1001)
<223>  n is a, c, g, or t

<220>
<221>  misc_feature
<222>  (1034)..(1035)
<223>  n is a, c, g, or t

<220>
<221>  misc_feature
<222>  (1037)..(1038)
<223>  n is a, c, g, or t

<220>
<221>  misc_feature
<222>  (1041)..(1041)
<223>  n is a, c, g, or t

<220>
<221>  misc_feature
<222>  (1043)..(1044)
<223>  n is a, c, g, or t

<220>
<221>  misc_feature
<222>  (1051)..(1052)
<223>  n is a, c, g, or t

<220>
<221>  misc_feature
<222>  (1054)..(1056)
<223>  n is a, c, g, or t

<220>
<221>  misc_feature
<222>  (1071)..(1072)
<223>  n is a, c, g, or t

<220>
<221>  misc_feature
<222>  (1075)..(1075)
<223>  n is a, c, g, or t

<220>
<221>  misc_feature
<222>  (1150)..(1150)
<223>  n is a, c, g, or t

<220>
<221>  misc_feature
<222>  (1152)..(1152)
<223>  n is a, c, g, or t

<220>
<221>  misc_feature
<222>  (1154)..(1154)
<223>  n is a, c, g, or t

<220>
<221>  misc_feature
<222>  (1169)..(1169)
<223>  n is a, c, g, or t

<220>
<221>  misc_feature
<222>  (1185)..(1185)
<223>  n is a, c, g, or t

<220>
<221>  misc_feature
<222>  (1289)..(1289)
<223>  n is a, c, g, or t

<220>
<221>  misc_feature
<222>  (1295)..(1295)
<223>  n is a, c, g, or t

<220>
<221>  misc_feature
<222>  (1304)..(1305)
<223>  n is a, c, g, or t

<220>
<221>  misc_feature
<222>  (1310)..(1310)
<223>  n is a, c, g, or t

<220>
<221>  misc_feature
<222>  (1339)..(1339)
<223>  n is a, c, g, or t

<220>
<221>  misc_feature
<222>  (1342)..(1342)
<223>  n is a, c, g, or t

<220>
<221>  misc_feature
<222>  (1359)..(1359)
<223>  n is a, c, g, or t

<220>
<221>  misc_feature
<222>  (1362)..(1362)
<223>  n is a, c, g, or t

<220>
<221>  misc_feature
<222>  (1368)..(1368)
<223>  n is a, c, g, or t

<220>
<221>  misc_feature
<222>  (1396)..(1396)

<223>  n is a, c, g, or t

<220>
<221>  misc_feature
<222>  (1468)..(1468)
<223>  n is a, c, g, or t

<220>
<221>  misc_feature
<222>  (1475)..(1476)
<223>  n is a, c, g, or t

<220>
<221>  misc_feature
<222>  (1482)..(1482)
<223>  n is a, c, g, or t

<220>
<221>  misc_feature
<222>  (1485)..(1487)
<223>  n is a, c, g, or t

<220>
<221>  misc_feature
<222>  (1492)..(1493)
<223>  n is a, c, g, or t

<220>
<221>  misc_feature
<222>  (1499)..(1499)
<223>  n is a, c, g, or t

<220>
<221>  misc_feature
<222>  (1506)..(1506)
<223>  n is a, c, g, or t

<220>
<221>  misc_feature
<222>  (1518)..(1521)
<223>  n is a, c, g, or t

<220>
<221>  misc_feature
<222>  (1524)..(1524)
<223>  n is a, c, g, or t

<400>  8
nnnnnnnnag agtnngatnn tggctcagga ngaacgctgg cggcgtgcct aatacatgca      60

agtcgaacgn nnnnnnnnnn nnnnnnnnnn nngcttgcnn nnnnnnnnnn nnnnnnnnnn     120

nnagnggcgg acgggtgagt aacacgtggg naacctgccc nnaagnnggg gataacantt     180

ggaaacagnt gctaataccg nataacnacn nnnancgcat gntnnnnnnn tnaaagntgg     240

cntnngctat cnctnnngga tggacccgcg gcgnattagc tagttggtga ggtaanggcn     300

naccaaggcn atgatncgta gccganntga gagnntnatc ggccacantg ggactgagac     360

acggccnnna ctcctacggg aggcagcagt agggaatctt ccacaatgga cgnaagnctg     420

atggagcaac gccgcgtgag tgaagaaggn tttcggntcg taaaactctg ttgttngaga     480

agaannnnng ngagagtaac tgntcnnnnn ntgacggtat nnaaccagaa agncacggct     540

aactacgtgc cagcagccgc ggtaatacgt aggtggcnag cgttntccgg atttattggg     600

cgtaaagcga gcgcaggcgg tnnnntaagt ctgatgtgaa agccnncggc tnaaccgnng 660

aagtgcatcg gaaactggnn nacttgagtg cagaagagga nagtggaact ccatgtgtag 720

cggtgnaatg cgtagatata tggaagaaca ccagtggcga aggcggctnt ctggtctgna 780

actgacgctg aggctcgaaa gcatgggtag cnaacaggat tagataccct ggtagtccat 840

gccgtaaacg atgantgcta ngtgttggag ggtttccgcc cttcagtgcn gcagctaacg 900

cattaagcan tccgcctggg gagtacgacc gcaaggttga aactcaaagg aattgacggg 960

ggccngcaca agcggtggag catgtggttt aattcgaagn nacgcgaaga accttaccag 1020

gtcttgacat cttnngnnaa ncnnagagat nngnnnttcc cttcggggac nnaangacag 1080

gtggtgcatg gttgtcgtca gctcgtgtcg tgagatgttg ggttaagtcc cgcaacgagc 1140

gcaacccttn tnantagttg ccagcattna gttgggcact ctagngagac tgccggtgac 1200

aaaccggagg aaggtgggga tgacgtcaaa tcatcatgcc ccttatgacc tgggctacac 1260

acgtgctaca atggacggta caacgagtng cgaanccgcg aggnnaagcn aatctcttaa 1320

agccgttctc agttcggant gnaggctgca actcgcctnc angaagtngg aatcgctagt 1380

aatcgcggat cagcangccg cggtgaatac gttcccgggc cttgtacaca ccgcccgtca 1440

caccatgaga gtttgtaaca cccaaagncg gtggnntaac cnttnnngga gnnagccgnc 1500

taaggnggga cagatgannn nggn 1524

<210> 9
<211> 1495
<212> DNA
<213> Artificial

<220>
<223> Lactococcus consensus 2


<220>
<221> misc_feature
<222> (1)..(2)
<223> n is a, c, g, or t

<220>
<221> misc_feature
<222> (69)..(69)
<223> n is a, c, g, or t

<220>
<221> misc_feature
<222> (71)..(76)
<223> n is a, c, g, or t

<220>
<221> misc_feature
<222> (78)..(79)
<223> n is a, c, g, or t

<220>
<221> misc_feature
<222> (86)..(87)
<223> n is a, c, g, or t

<220>

<221>  misc_feature
<222>  (89)..(91)
<223>  n is a, c, g, or t

<220>
<221>  misc_feature
<222>  (93)..(94)
<223>  n is a, c, g, or t

<220>
<221>  misc_feature
<222>  (98)..(98)
<223>  n is a, c, g, or t

<220>
<221>  misc_feature
<222>  (128)..(128)
<223>  n is a, c, g, or t

<220>
<221>  misc_feature
<222>  (138)..(138)
<223>  n is a, c, g, or t

<220>
<221>  misc_feature
<222>  (140)..(140)
<223>  n is a, c, g, or t

<220>
<221>  misc_feature
<222>  (154)..(154)
<223>  n is a, c, g, or t

<220>
<221>  misc_feature
<222>  (167)..(167)
<223>  n is a, c, g, or t

<220>
<221>  misc_feature
<222>  (187)..(187)
<223>  n is a, c, g, or t

<220>
<221>  misc_feature
<222>  (202)..(202)
<223>  n is a, c, g, or t

<220>
<221>  misc_feature
<222>  (204)..(205)
<223>  n is a, c, g, or t

<220>
<221>  misc_feature
<222>  (230)..(230)
<223>  n is a, c, g, or t

<220>
<221>  misc_feature
<222>  (232)..(232)
<223>  n is a, c, g, or t

<220>
<221>  misc_feature
<222>  (274)..(274)
<223>  n is a, c, g, or t

<220>
<221>  misc_feature
<222>  (276)..(276)
<223>  n is a, c, g, or t

<220>
<221>  misc_feature
<222>  (343)..(343)
<223>  n is a, c, g, or t

<220>
<221>  misc_feature
<222>  (554)..(554)
<223>  n is a, c, g, or t

<220>
<221>  misc_feature
<222>  (773)..(773)
<223>  n is a, c, g, or t

<220>
<221>  misc_feature
<222>  (837)..(837)
<223>  n is a, c, g, or t

<220>
<221>  misc_feature
<222>  (863)..(863)
<223>  n is a, c, g, or t

<220>
<221>  misc_feature
<222>  (939)..(939)
<223>  n is a, c, g, or t

<220>
<221>  misc_feature
<222>  (974)..(974)
<223>  n is a, c, g, or t

<220>
<221>  misc_feature
<222>  (1175)..(1175)
<223>  n is a, c, g, or t

<220>
<221>  misc_feature
<222>  (1266)..(1266)
<223>  n is a, c, g, or t

<220>
<221>  misc_feature
<222>  (1269)..(1269)
<223>  n is a, c, g, or t

<220>
<221>  misc_feature
<222>  (1280)..(1280)
<223>  n is a, c, g, or t

<220>
<221>  misc_feature
<222>  (1491)..(1492)
<223>  n is a, c, g, or t

<400>  9
nntgagagtt tgatcctggc tcaggacgaa cgctggcggc gtgcctaata catgcaagtt      60

gagcgcttna nnnnnngnng cttgcnncnn ntnngganga gcagcgaacg ggtgagtaac 120
gcgtgggnaa tctgcctntn agcgggggac aacntttgga aacgaangct aataccgcat 180
aacaatntta aacacatgtt tnanntttga aagaagcaat tgcttcactn anagatgatc 240
ccgcgttgta ttagctagtt ggtagtgtaa aggncnacca aggcgatgat acatagccga 300
cctgagaggg tgatcggcca cattgggact gagacacggc ccnaactcct acgggaggca 360
gcagtaggga atcttcggca atggacgaaa gtctgaccga gcaacgccgc gtgagtgaag 420
aaggttttcg gatcgtaaaa ctctgttgtt agagaagaac gttggtgaga gtggaaagtt 480
catcaagtga cggtatctaa ccagaaaggg acggctaact acgtgccagc agccgcggta 540
atacgtaggt cccnagcgtt gtccggattt attgggcgta aagcgagcgc aggtggttta 600
ttaagtctga tgtaaaaggc agtggctcaa ccattgtgtg cattggaaac tggtagactt 660
gagtgcagga gaggagagtg gaattccatg tgtagcggtg aaatgcgtag atatatggag 720
gaacaccggt ggcgaaagcg gctctctggc ctgtaactga cactgaggct cgnaagcgtg 780
gggagcaaac aggattagat accctggtag tccacgccgt aaacgatgag tgctagntgt 840
agggagctat aagttctctg tancgcagct aacgcattaa gcactccgcc tggggagtac 900
gaccgcaagg ttgaaactca aaggaattga cgggggccng cacaagcggt ggagcatgtg 960
gtttaattcg aagnaacgcg aagaacctta ccaggtcttg acatactcgt gctattccta 1020
gagataggaa gttccttcgg gacacgggat acaggtggtg catggttgtc gtcagctcgt 1080
gtcgtgagat gttgggttaa gtcccgcaac gagcgcaacc cctattgtta gttgccatca 1140
ttaagttggg cactctagcg agactgccgg tgatnaaccg gaggaaggtg gggatgacgt 1200
caaatcatca tgccccttat gacctgggct acacacgtgc tacaatggat ggtacaacga 1260
gtcgcnagnc agtgatgttn agctaatctc ttaaaaccaa tctcagttcg gattgtaggc 1320
tgcaactcgc ctacatgaag tcggaatcgc tagtaatcgc ggatcagcac gccgcggtga 1380
atacgttccc gggccttgta cacaccgccc gtcacaccac gggagttggg agtacccaaa 1440
gtaggttgcc taaccgcaag gagggcgctt cctaaggtaa gaccgatgac nnggg 1495

<210> 10
<211> 1481
<212> DNA
<213> Artificial

<220>
<223> Staphylococcus consensus 2

<220>
<221> misc_feature
<222> (1)..(4)
<223> n is a, c, g, or t

<220>
<221> misc_feature
<222> (54)..(55)
<223> n is a, c, g, or t

<220>
<221>  misc_feature
<222>  (70)..(70)
<223>  n is a, c, g, or t

<220>
<221>  misc_feature
<222>  (126)..(126)
<223>  n is a, c, g, or t

<220>
<221>  misc_feature
<222>  (133)..(133)
<223>  n is a, c, g, or t

<220>
<221>  misc_feature
<222>  (145)..(145)
<223>  n is a, c, g, or t

<220>
<221>  misc_feature
<222>  (152)..(152)
<223>  n is a, c, g, or t

<220>
<221>  misc_feature
<222>  (161)..(161)
<223>  n is a, c, g, or t

<220>
<221>  misc_feature
<222>  (164)..(164)
<223>  n is a, c, g, or t

<220>
<221>  misc_feature
<222>  (181)..(181)
<223>  n is a, c, g, or t

<220>
<221>  misc_feature
<222>  (183)..(183)
<223>  n is a, c, g, or t

<220>
<221>  misc_feature
<222>  (197)..(197)
<223>  n is a, c, g, or t

<220>
<221>  misc_feature
<222>  (462)..(462)
<223>  n is a, c, g, or t

<220>
<221>  misc_feature
<222>  (993)..(994)
<223>  n is a, c, g, or t

<220>
<221>  misc_feature
<222>  (1009)..(1010)
<223>  n is a, c, g, or t

<220>
<221>  misc_feature

<222>  (1438)..(1438)
<223>  n is a, c, g, or t

<220>
<221>  misc_feature
<222>  (1442)..(1443)
<223>  n is a, c, g, or t

<220>
<221>  misc_feature
<222>  (1481)..(1481)
<223>  n is a, c, g, or t

<400>  10
nnnnaggatg aacgctggcg gcgtgcctaa tacatgcaag tcgagcgaac agannaggag      60

cttgctcctn tgacgttagc ggcggacggg tgagtaacac gtgggtaacc tacctataag     120

actggnataa ctncgggaaa ccggngctaa tnccggataa natnttgaac cgcatggttc     180

nanagtgaaa gacggtnttg ctgtcactta tagatggacc cgcgccgtat tagctagttg     240

gtaaggtaac ggcttaccaa ggcaacgata cgtagccgac ctgagagggt gatcggccac     300

actggaactg agacacggtc cagactccta cgggaggcag cagtagggaa tcttccgcaa     360

tgggcgaaag cctgacggag caacgccgcg tgagtgatga aggtcttcgg atcgtaaaac     420

tctgttatta gggaagaaca aatgtgtaag taactgtgca cntcttgacg gtacctaatc     480

agaaagccac ggctaactac gtgccagcag ccgcggtaat acgtaggtgg caagcgttat     540

ccggaattat tgggcgtaaa gcgcgcgtag gcggtttttt aagtctgatg tgaaagccca     600

cggctcaacc gtggagggtc attggaaact ggaaaacttg agtgcagaag aggaaagtgg     660

aattccatgt gtagcggtga aatgcgcaga gatatggagg aacaccagtg gcgaaggcga     720

ctttctggtc tgtaactgac gctgatgtgc gaaagcgtgg ggatcaaaca ggattagata     780

ccctggtagt ccacgccgta aacgatgagt gctaagtgtt agggggtttc cgccccttag     840

tgctgcagct aacgcattaa gcactccgcc tggggagtac gaccgcaagg ttgaaactca     900

aaggaattga cggggacccg cacaagcggt ggagcatgtg gtttaattcg aagcaacgcg     960

aagaacctta ccaaatcttg acatcctttg acnnctctag agatagagnn ttccccttcg    1020

ggggacaaag tgacaggtgg tgcatggttg tcgtcagctc gtgtcgtgag atgttgggtt    1080

aagtcccgca acgagcgcaa cccttaagct tagttgccat cattaagttg ggcactctaa    1140

gttgactgcc ggtgacaaac cggaggaagg tggggatgac gtcaaatcat catgcccctt    1200

atgatttggg ctacacacgt gctacaatgg acaatacaaa gggcagcgaa accgcgaggt    1260

caagcaaatc ccataaagtt gttctcagtt cggattgtag tctgcaactc gactacatga    1320

agctggaatc gctagtaatc gtagatcagc atgctacggt gaatacgttc ccgggtcttg    1380

tacacaccgc ccgtcacacc acgagagttt gtaacacccg aagccggtgg agtaaccntt    1440

tnnggagcta gccgtcgaag gtgggacaaa tgattggggt n                         1481

<210>  11
<211>  20
<212>  DNA

<213>  Artificial

<220>
<223>  primer

<400>  11
ccattgtarc aygygtgwag                                                    20


<210>  12
<211>  23
<212>  DNA
<213>  Artificial

<220>
<223>  primer

<400>  12
tcgtgyyrts arrtgtyrgs tya                                                23


<210>  13
<211>  22
<212>  DNA
<213>  Artificial

<220>
<223>  primer 2

<400>  13
accttacctg ggcttgacat gt                                                 22


<210>  14
<211>  21
<212>  DNA
<213>  Artificial

<220>
<223>  primer 8

<400>  14
cgcgaagaac cttaccaaat c                                                  21


<210>  15
<211>  20
<212>  DNA
<213>  Artificial

<220>
<223>  primer 1

<400>  15
ttacccgggc ttaaattgca                                                    20


<210>  16
<211>  21
<212>  DNA
<213>  Artificial

<220>
<223>  primer 3

<400>  16
agaacttagc agagatgctt t                                                  21

<210>  17
<211>  21
<212>  DNA
<213>  Artificial

<220>
<223>  primer 4

<400>  17
accactctag agatagagct t                                                21

<210>  18
<211>  20
<212>  DNA
<213>  Artificial

<220>
<223>  primer 5

<400>  18
tcttgacatc cacggaagtt                                                  20

<210>  19
<211>  21
<212>  DNA
<213>  Artificial

<220>
<223>  primer 6

<400>  19
ccttaccagg tcttgacatc t                                                21

<210>  20
<211>  22
<212>  DNA
<213>  Artificial

<220>
<223>  primer 7

<400>  20
ctattcctag agataggaag tt                                               22